# EUROPEAN PATENT SPECIFICATION

(11) **EP 1 776 359 B1**
(45) Date of publication and mention of the grant of the patent: **24.08.2011**
(21) Application number: 05761185.7
(22) Date of filing: 11.07.2005
(51) Int. Cl.: C07D 413/14, C07D 413/12, A61K 31/538

(54) **PIPERIDINE DERIVATIVES AS RENIN INHIBITORS**
PIPERIDINDERIVATE ALS RENIN-INHIBITOREN
DÉRIVÉS DE LA PIPÉRIDINE COMME INHIBITEURS DE LA RENINE

(30) Priority: 09.07.2004 CH 115804
(43) Date of publication of application: 25.04.2007
(73) Proprietor: Novartis AG, 4056 Basel (CH)
(72) Inventor: HEROLD, Peter, CH-4055 Basel (CH); MAH, Robert, CH-4132 Muttenz (CH); STUTZ, Stefan, CH-4053 Basel (CH); STOJANOVIC, Aleksandar, CH-4055 Basel (CH); TSCHINKE, Vincenzo, CH-4102 Binningen (CH); JOTTERAND, Nathalie, CH-4053 Basel (CH); BEHNKE, Dirk, 79639 Grenzach-Wyhlen (DE)
(86) International application number: PCT/EP2005/053306
(87) International publication number: WO 2006/005741

(56) References cited:
- WO-A-00/64887
- WO-A-97/09311
- WO-A-02/088101
- WO-A-2004/089903
- MARKI H P ET AL: "Piperidine renin inhibitors: from leads to drug candidates" 2001, IL FARMACO, ROME, IT, PAGE(S) 21-27 , XP002317172 ISSN: 0014-827X page 24; examples 19-23

## Description

The present invention relates to novel substituted piperidines, to processes for their preparation and to the use of the compounds as medicines, in particular as renin inhibitors.

Piperidine derivatives for use as medicines are known, for example from WO97/09311. However, especially with regard to renin inhibition, there is still a need for highly potent active ingredients, In this context, the improvement of the pharmacokinetic properties is at the forefront. These properties directed towards better bioavailability are, for example, absorption, metabolic stability, solubility or lipophilicity.

The invention therefore provides
a compound of the formula (1) 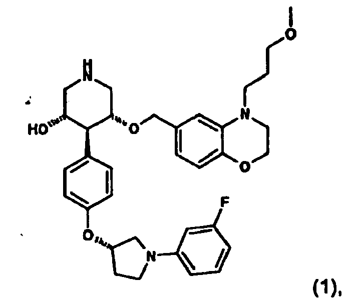 a compound of the formula (2) 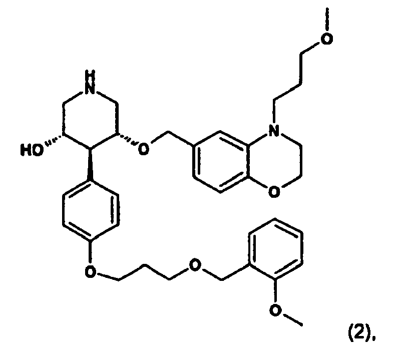 a compound of the formula (3) 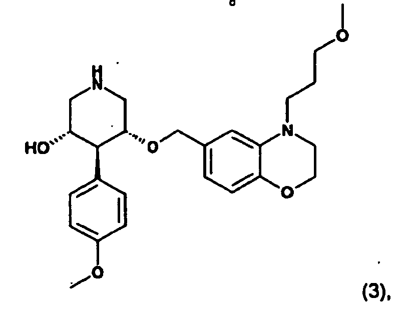 a compound of the formula (4) 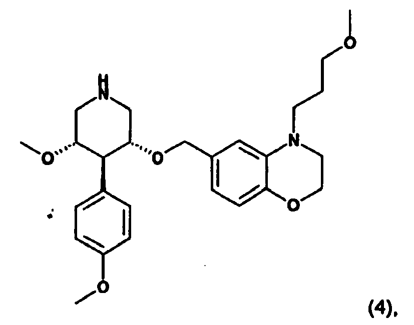 a compound of the formula (5) 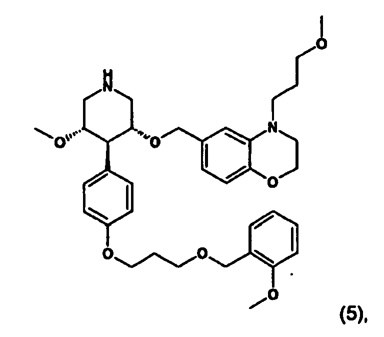 a compound of the formula (6) 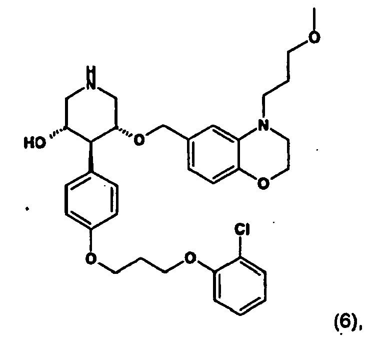 a compound of the formula (7) 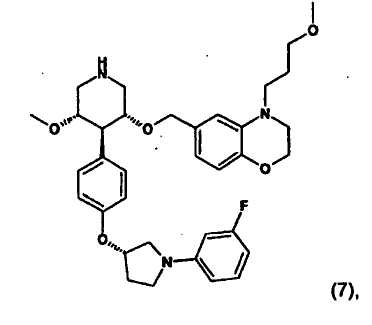 a compound of the formula (8) 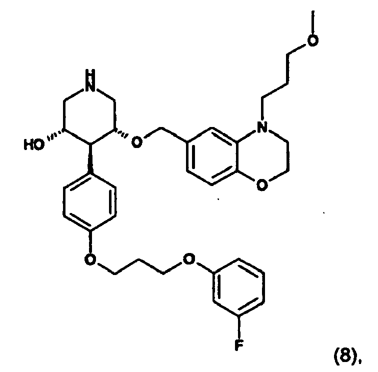 a compound of the formula (9) 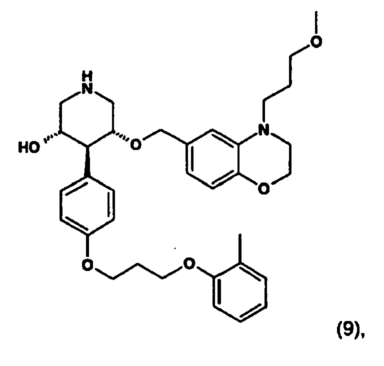 a compound of the formula (10) 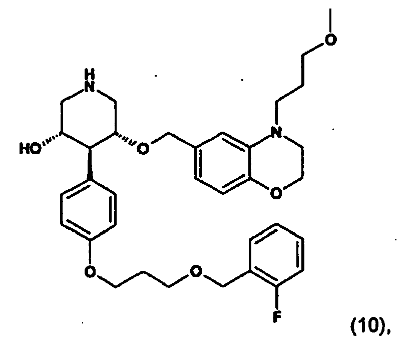 a compound of the formula (11) 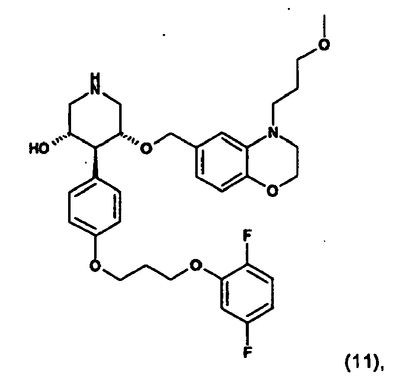 a compound of the formula (12) 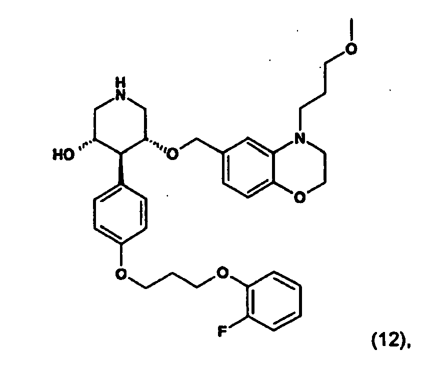 a compound of the formula (13) 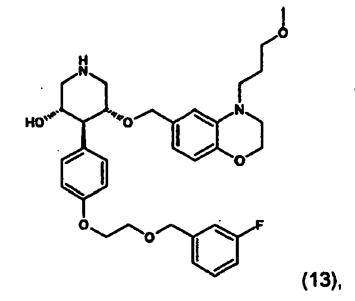 a compound of the formula (14) 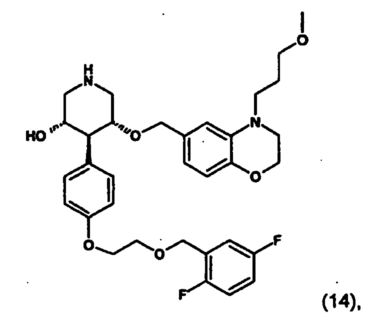 a compound of the formula (15) 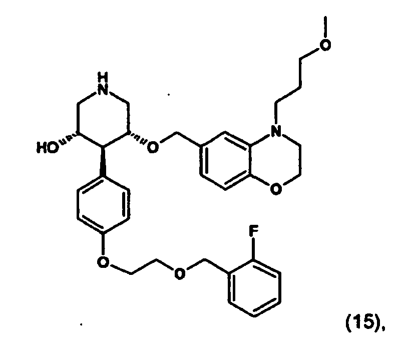 a compound of the formula (16) 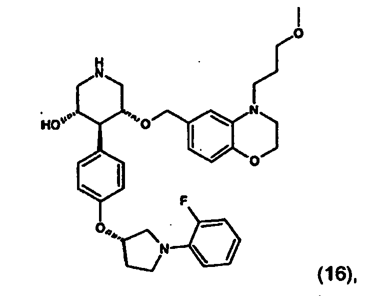 a compound of the formula (17) 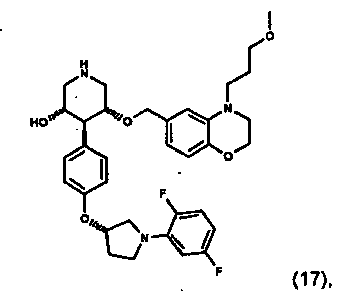 a compound of the formula (18) 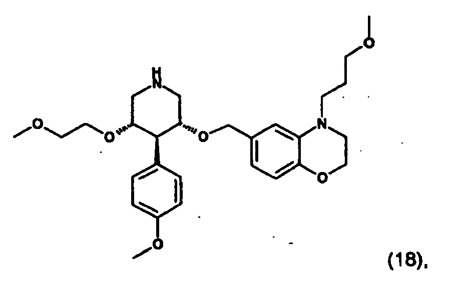 a compound of the formula (19) 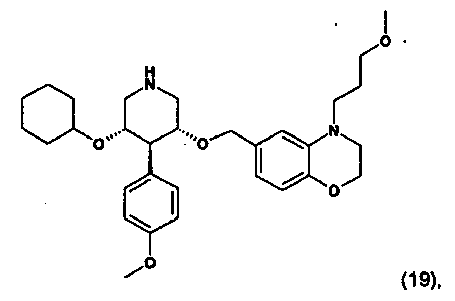 a compound of the formula (20) 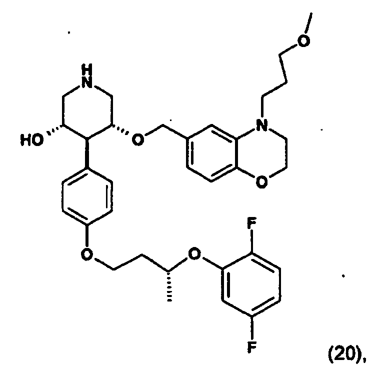 a compound of the formula (21) 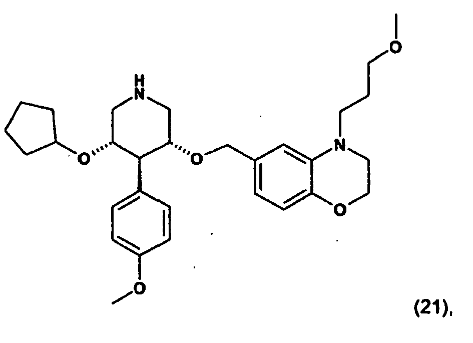 a compound of the formula (22) 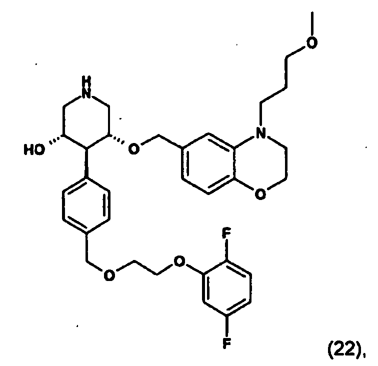 a compound of the formula (23) 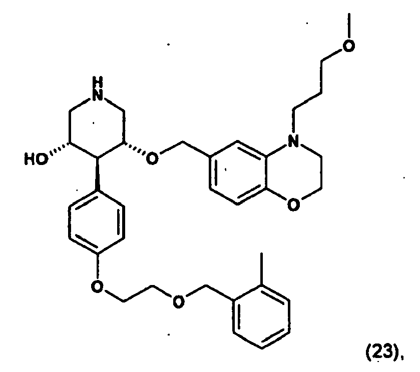 a compound of the formula (24) 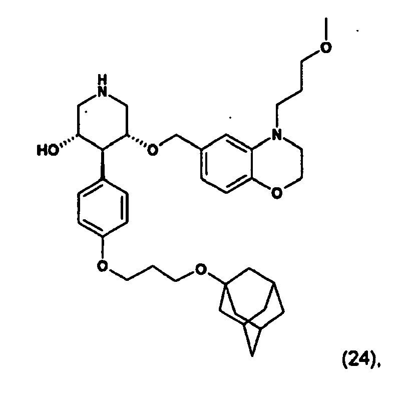 a compound of the formula (25) 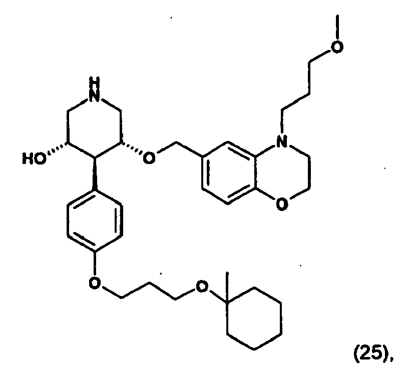 a compound of the formula (26) 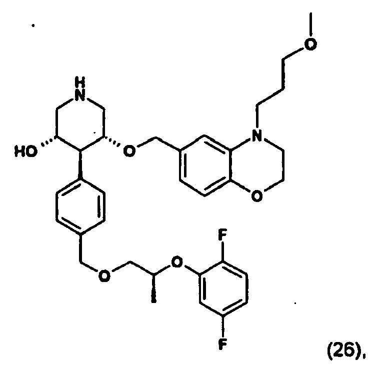 a compound of the formula (27) 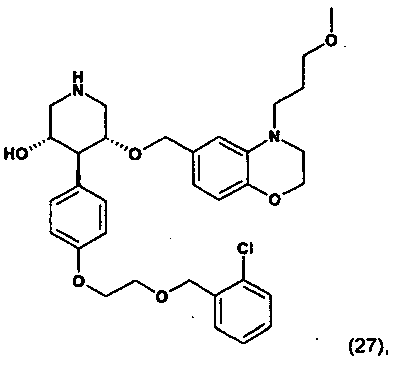 a compound of the formula (28) 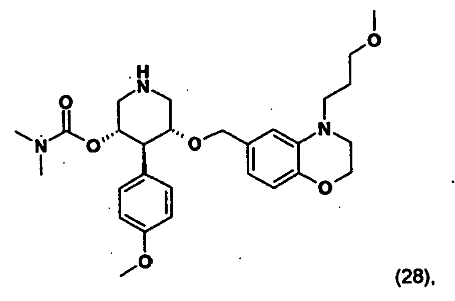 a compound of the formula (29) 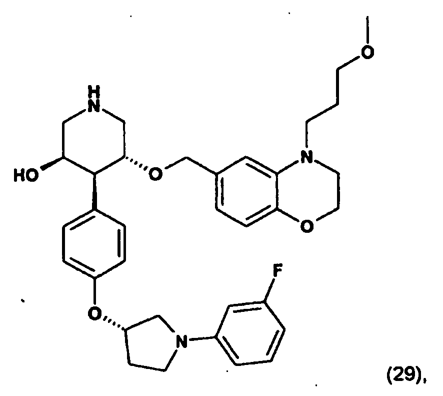 a compound of the formula (30) 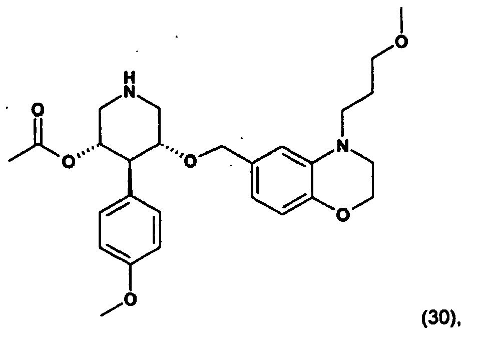 a compound of the formula (31) 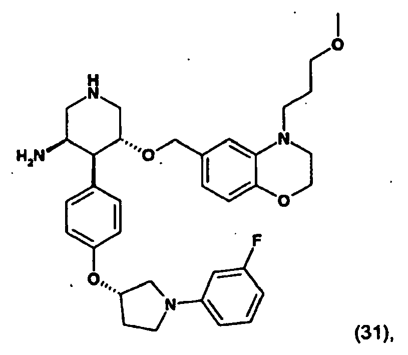 a compound of the formula (32) 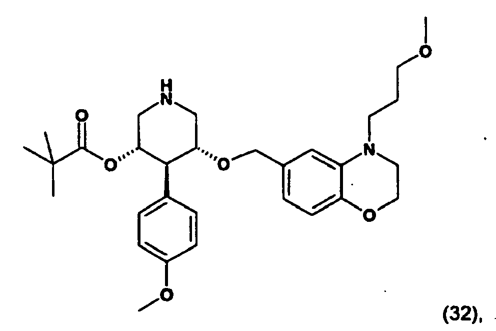 a compound of the formula (33) 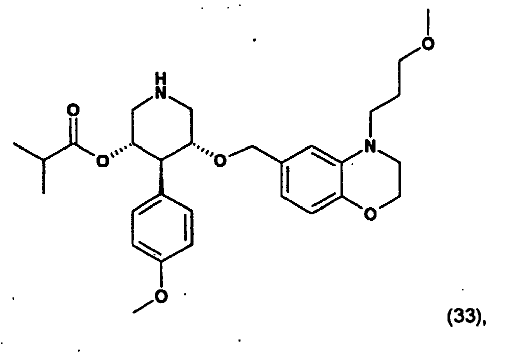 a compound of the formula (34) 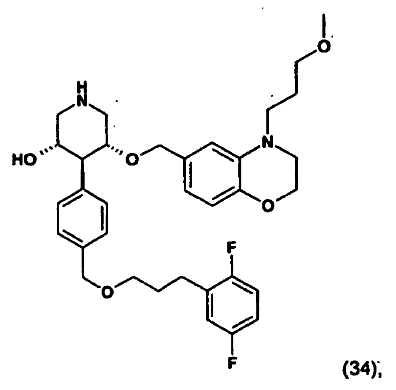 a compound of the formula (35) 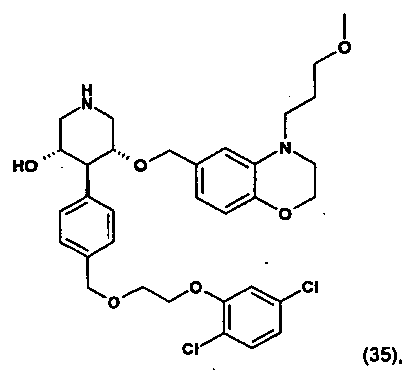 a compound of the formula (36) 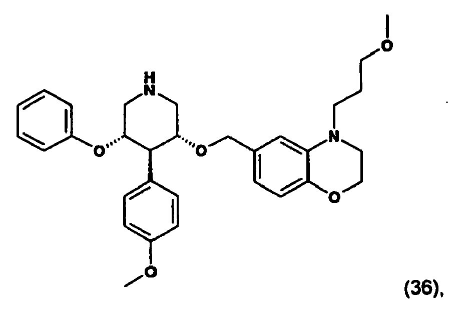 a compound of the formula (37) 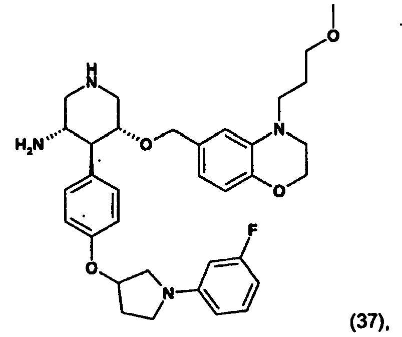 a compound of the formula (38) 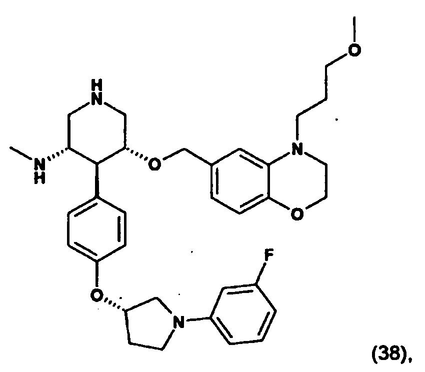 a compound of the formula (39) 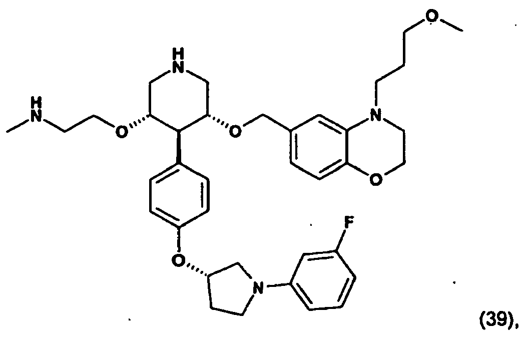 a compound of the formula (40) 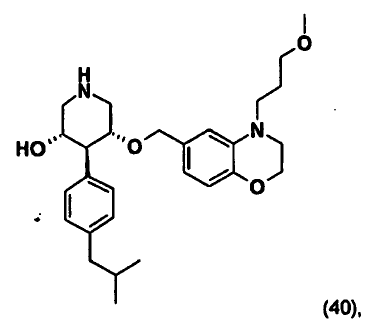 a compound of the formule (41) 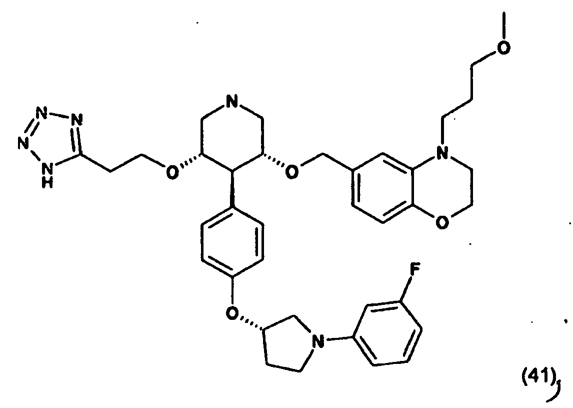 or a pharmaceutically acceptable salt thereof

The compounds of the invention have at least three asymmetric carbon atoms and may therefore be present in the form of optically pure diastereomers, diastereomer mixtures, diastereomeric racemates, mixtures of diastereomeric racemates or as meso compounds. The invention encompasses all of theses forms. Diastereomer mixtures, diastereomeric racemates or mixtures of diastereomeric racemates may be separated by customary methods, for example by column chromatography, thin-layer chromatography, HPLC and the like.

The expression "pharmaceutically acceptable salts" encompasses salts with inorganic or organic acids such as hydrochloric acid, hydrobromic acid, nitric acid, sulphuric acid, phosphoric acid, citric acid, formic acid, maleic acid, acetic acid, succinic acid, tartaric acid, methanesulphonic acid, p-toluenesulphonic acid and the like.

The compounds of the invention may be prepared in an analogous manner to the preparation processes known from the literature. Similar preparation processes are described, for example, In WO 97/09311. Details of the specific preparation variants can be taken from the examples.

The compounds of the invention may also be prepared in optically pure form. The separation into antipodes may be effected by methods known per se, either preferably at a synthetically early stage by salt formation with an optically active acid, for example (+)- or (-)-mandelic acid, and separation of the diastereomeric salts by fractional crystallization, or preferably at a rather late stage by derivatization with a chiral auxiliary building block, for example (+)- or (-)-camphanoyl chloride, and separation of the diastereomeric products by chromatography and/or crystallization and subsequent cleavage of the bond to the chiral auxiliary. To determine the absolute configuration of the piperidine present, the pure diastereomeric salts and derivatives may be analysed with common spectroscopic methods, of which X-ray spectroscopy on single crystals constitutes a particularly suitable method.

The compounds of the invention also include those compounds in which one or more atoms are replaced by their stable, non-radioactive isotopes; for example, a hydrogen atom by deuterium.

The compounds of the invention the pharmaceutically acceptable salts thereof have inhibiting action on the natural enzyme renin. The latter passes from the kidneys into the blood and therein brings about the cleavage of angiotensinogen to form the decapeptide angiotensin I which is then cleaved in the lung, the kidneys and other organs to the octapeptide angiotensin II. Angiotensin II increases the blood pressure both directly by arterial constriction and indirectly by the release of the hormone aldosterone which inhibits the release of the sodium ion from the adrenal glands, which is associated with a rise in the extracellular liquid volume. This rise can be attributed to the action of angiotensin II itself or of the heptapeptide angiotensin III formed therefrom as a cleavage product. Inhibitors of the enzymatic activity of renin bring about a reduction in the formation of angiotensin I and, as a consequence thereof, the formation of a smaller amount of angiotensin II. The reduced concentration of this active peptide hormone is the immediate cause of the hypotensive action of renin inhibitors.

One experimental method of detecting the action of renin inhibitors is by means of in vitro tests, in which the reduction of the formation of angiotensin I in different systems (human plasma, purified human renin together with synthetic or natural renin substrate) is measured. One in vitro test which is used is the one according to Nussberger et al. (1987) J. Cardiovascular Pharmacol., Vol. 9, p. 39-44 which follows. This test measures the formation of angiotensin I in human plasma. The amount of angiotensin I formed is determined in a subsequent radioimmunoassay. Which action inhibitors have on the formation of angiotensin I is tested in this system by the addition of different concentrations of these substances. The IC₅₀ refers to that concentration of the particular inhibitor which reduces the formation of angiotensin I by 50%. The compounds of the present invention exhibit inhibiting actions in the in vitro systems at minimum concentrations of about 10⁻⁶ to about 10⁻¹⁰ mol/l.

In salt-depleted animals, renin inhibitors bring about a blood pressure decrease. Human renin differs from renin of other species. To test inhibitors of human renin, primates (marmosets, Callithrixjacchus) are used, because human renin and primate renin are substantially homologous in the enzymatically active region. One in vivo test which is used is as follows: the test compounds are tested on normotensive marmosets of both genders and having a body weight of about 350 g which are conscious, able to move freely and in their normal cages. Blood pressure and heart rate are measured using a catheter in the descending aorta and recorded radiometrically. The endogenous release of renin is stimulated by the combination of a 1-week low-salt diet with a single intramuscular injection of furosemide (5-aminosulphonyl)4-chloro-2-[(2-furanylmethyl)amino]benzoic acid) (5 mg/kg). 16 hours after the injection of furosemide, the test substances are administered either directly into the femoral artery by means of an injection cannula or into the stomach by gavage as a suspension or solution, and their effect on blood pressure and heart rate is evaluated. The compounds of the present invention effectively reduce blood pressure in the in vivo test described at doses of about 0.003 to about 0.3 mg/kg i.v. and at doses of about 0.3 to about 30 mg/kg p.o.

The compounds of the invention and the pharmaceutically acceptable salts thereof, may find use as medicines, for example in the form of pharmaceutical compositions. The pharmaceutical compositions may be administered enterally, such as orally, for example in the form of tablets, film-coated tablets, sugar-coated tablets, hard and soft gelatin capsules, solutions, emulsions or suspensions, nasally, for example in the form of nasal sprays, rectally, for example in the form of suppositories, or transdermally, for example in the form of ointments or patches. The administration may also be parenteral, such as intramuscular or intravenous, for example in the form of injection solutions.

To prepare tablets, film-coated tablets, sugar-coated tablets and hard gelatin capsules, the compounds of the invention and pharmaceutically acceptable salts thereof may be processed with pharmaceutically inert, inorganic or organic excipients. Such excipients used, for example for tablets, film-coated tablets and hard gelatin capsules, may be lactose, com starch, or derivatives thereof, talc, stearic acid or salts thereof etc.

Suitable excipients for soft gelatin capsules are, for example, vegetable oils, waxes, fats, semisolid and liquid polyols, etc.

Suitable excipients for preparing solutions and syrups are, for example, water, polyols, sucrose, invert sugar, glucose, etc.

Suitable excipients for injection solutions are, for example, water, alcohols, polyols, glycerol, vegetable oils, bile acids, lecithin, etc.

Suitable excipients for suppositories are, for example, natural or hardened oils, waxes, fats, semisolid or liquid polyols, etc.

The pharmaceutical compositions may additionally also comprise preservatives, solubilizers, viscosity-increasing substances, stabilizers, wetting agents, emulsifiers, sweeteners, colorants, flavourings, salts for altering the osmotic pressure, buffers, coatings or antioxidants. They may also comprise other therapeutically valuable substances.

The present invention further provides for the use of the compounds of the invention and the pharmaceutically acceptable salts thereof, in the manufacture of a medicament for the treatment or prevention of hypertension and heart failure, and also glaucoma, myocardial Infarction, kidney failure and restenoses.

The compounds of the invention and the pharmaceutically acceptable salts thereof, may also be administered in combination with one or more agents having cardiovascular action, for example α- and β-blockers such as phentolamine, phenoxybenzamine, prazosin, terazosin, tolazine, atenolol, metoprolol, nadolol, propranolol, timolol, carteolol etc.; vasodilators such as hydralazine, minoxidil, diazoxide, nitroprusside, flosequinan etc.; calcium antagonists such as amrinone, bencyclan, diltiazem, fendiline, flunarizine, nicardipine, nimodipine, perhexilene, verapamil, gallopamil, nifedipine etc.; ACE inhibitors such as cilazapril, captopril, enalapril, lisinopril etc.; potassium activators such as pinacidil; anti-serotoninergics such as ketanserin; thromboxane-synthetase inhibitors; neutral endopeptidase inhibitors (NEP inhibitors); angiotensin II antagonists; and also diuretics such as hydrochlorothiazide, chlorothiazide, acetazolamide, amiloride, bumetanide, benzthiazide, ethacrynic add, furosemide, indacrinone, metolazone, spironolactone, triamteren, chlorthalidone etc.; sympatholytics such as methyldopa, clonidine, guanabenz, reserpine; and other agents which are suitable for the treatment of hypertension, heart failure or vascular diseases in humans and animals which are associated with diabetes or renal disorders such as acute or chronic renal failure. Such combinations may be employed separately or in preparations which comprise a plurality of components.

Further substances which can be used in combination with the compounds of the invention are the compounds of classes (i) to (ix) on page 1 of WO 02/40007 (and also the preferences and examples further listed therein) and the substances specified on pages 20 and 21 of WO 03/027091.

The dose may vary within wide limits and has of course to be adapted to the individual circumstances in each individual case. In general, for oral administration, a daily dose of about 3 mg to about 3 g, preferably about 10 mg to about 1 g, for example about 300 mg, per adult (70 kg), divided into preferably 1-3 individual doses which may, for example, be of equal size, may be appropriate, although the upper limit specified may also be exceeded if this should be found to be appropriate; typically, children receive a lower dose according to their age and body weight.

The examples which follow illustrate the present invention. All temperatures are reported in degrees Celsius, pressures in mbar. Unless stated otherwise, the reactions take place at room temperature. The abbreviation "Rf = xx (A)" means, for example, that the Rf value xx is determined in the solvent system A. The ratio of solvents relative to one another is always reported in parts by volume. Chemical names for end products and intermediates were generated with the aid of the program AutoNom 2000 (automatic nomenclature). Unless stated otherwise, the absolute stereochemistry of the 3-hydroxy(or alkoxy)4-phenyl-5-alkoxypiperidine unit is (3S,4S,5R) (or 3S,4R,5R, depending on the 3-alkoxy group).

| **No.** | **Structure** | **Appearance** | **R_{f}(System)** | **Rt (Method)** |
|---|---|---|---|---|
| 1 | 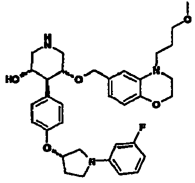 | colourless solid | 0.29 (A) | 4.28 (I) |
| 2 | 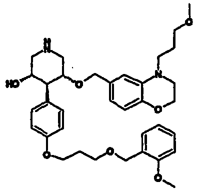 | colourless oil | 0.15 (A) | 4.14 (I) |
| 3 | 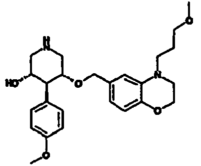 | yellowish oil | 0.05 (C) | 3.29 (I) |
| 4 | 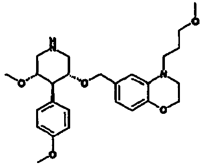 | yellowish oil | 0.15 (C) | 3.63 (I) |
| 5 | 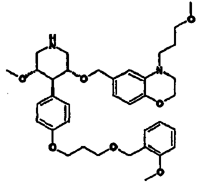 | colourless oil | 0.55 (A) | 4.43 (I) |
| 6 | 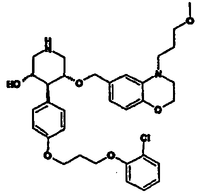 | orange oil | 0.06 (C) | 4.49 (I) |
| 7 | 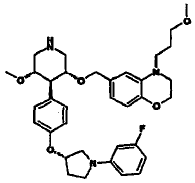 | colorless oil | 0.39 (A) | 4.56 (I) |
| 8 | 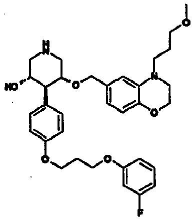 | orange oil | 0.20 (C) | 4.40 (I) |
| 9 | 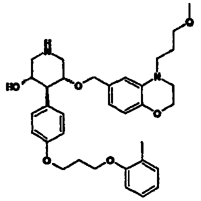 | yellowish oil | 0.18 (C) | 4.63 (I) |
| 10 | 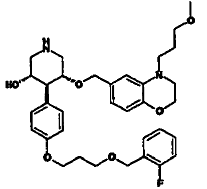 | yellowish oil | 0.15 (C) | 4.30 (I) |
| 11 | 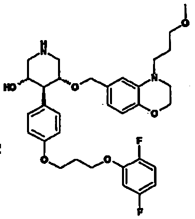 | yellowish oil | 0.15 (C) | 4.33 (I) |
| 12 | 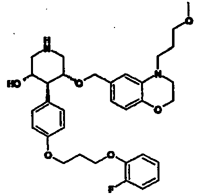 | yellowish oil | 0.15 (C) | 4.28 (I) |
| 13 | 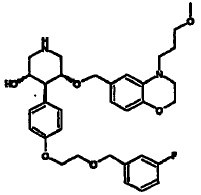 | yellowish oil | 0.11 (A) | 3.99 (I) |
| 14 | 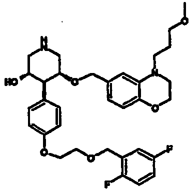 | yellowish oil | 0.14 (A) | 4.04 (I) |
| 15 | 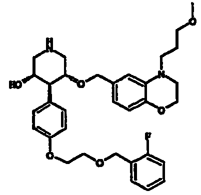 | yellowish oil | 0.15 (A) | 3.99 (I) |
| 16 | 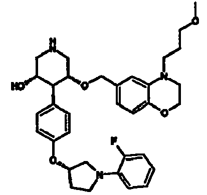 | colourless wax | 0.25 (A) | 4.21 (I) |
| 17 | 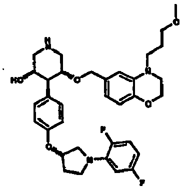 | colourless glass | 0.29 (A) | 4.35 (I) |
| 18 | 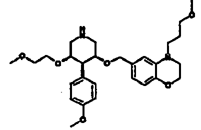 | colourless oil | 0.22 (C) | 3.50 (I) |
| 19 | 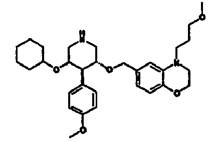 | yellow oil | 0.39 (C) | 4.19 (I) |
| 20 | 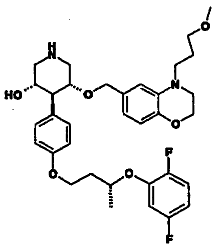 | colourless oil | 0.09 (C) | 4.52 (I) |
| 21 | 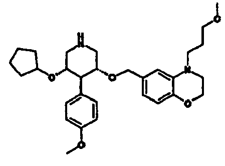 | yellow oil | 0.21 (C) | 4.04 (I) |
| 22 | 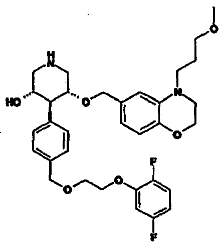 | colourless solid | 0.10 (C) | 4.18 (I) |
| 23 | 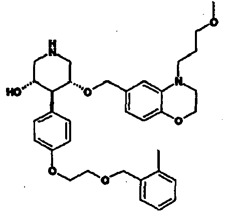 | pale yellow solid | 0.30 (A) | 4.13 (I) |
| 24 | 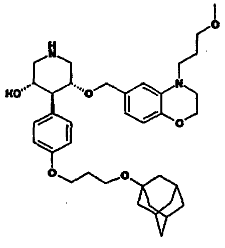 | light yellow oil | 0.09 (A) | 5.14 (1) |
| 25 | 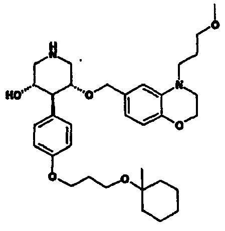 | light yellow oil | 0.08 (A) | 4.05 (I) |
| 26 | 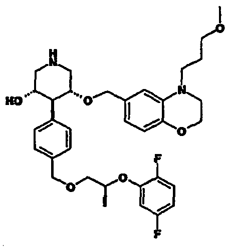 | light yellow oil | 0.19 (C) | 4.34 (I) |
| 27 | 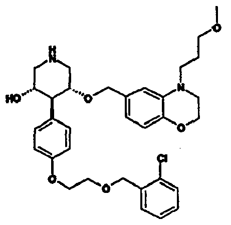 | light yellow solid | 0.17 (A) | 4.29 (I) |
| 28 | 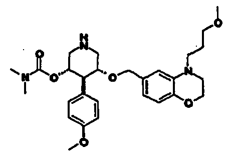 | yellow oil | 0.28 (A) | 3.67 (I) |
| 29 | 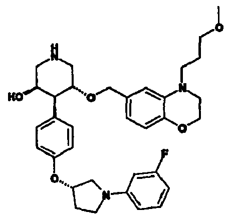 | yellow wax | 0.19 (A) | 4.50 (I) |
| 30 | 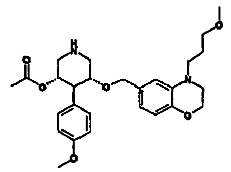 | colourless solid | 0.18 (C) | 3.65 (I) |
| 31 | 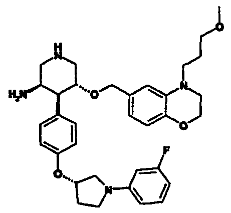 | beige solid | 0.13 (A) | 3.89 (I) |
| 32 | 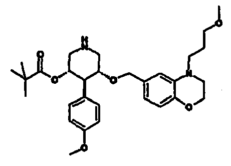 | beige solid | 0.25 (C) | 4.25 (I) |
| 33 | 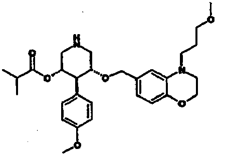 | light yellow oil | 0.28 (C) | 4.05 (I) |
| 34 | 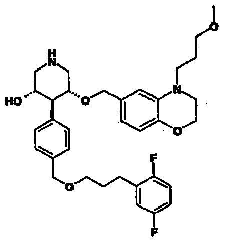 | colourless oil | 0.13 (C) | 4.49 (I) |
| 35 | 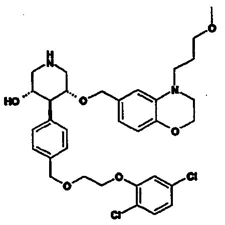 | colourless solid | 0.33 (C) | 4.64 (I) |
| 36 | 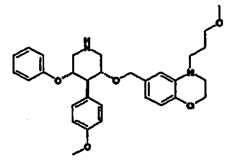 | colourless solid | 0.43 (A) | 4.14 (I) |
| 37 | 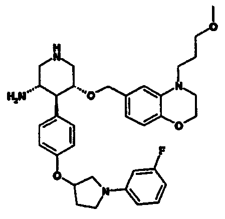 | beige solid | 0.13 (A) | 3.89 (I) |
| 38 | 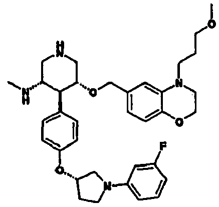 | light yellow solid | 0.28 (A) | 3.96 (I) |
| 39 | 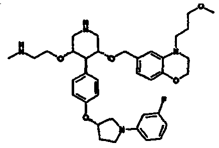 | light red oil | 0.10 (A) | 3.99 (I) |
| 40 | 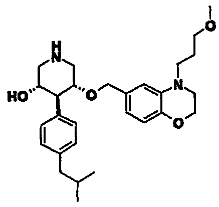 | colourless oil | 0.22 (A) | 4.28 (I) |
| 41 | 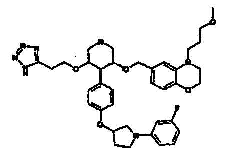 | yellowish foam | 0.09 (K) | 4.26 (I) |

Thin-layer chromatography eluent systems:
A Dichloromethane-methanol-25% conc. ammonia = 200:20:1
B Dichloromethane-methanol-25% conc. ammonia = 200:20:0.5
C Dichloromethane-methanol-25% conc. ammonia = 200:10:1
D Dichtoromethane-methanol-25% conc. ammonia = 90:10:1
E Dichloromethane-methanol-water-conc. acetic acid = 750:270:50:5
F Dichloromethane-methanol =1:4
G Dichloromethane-methanol-25% conc. ammonia = 200:5:1
H Dichloromethane-methanol = 9:1
I Dichloromethane-methanol-25% conc. ammonia = 40:10:1
J Dichloromethane-methanol-25% conc. ammonia = 80:10:1
K Dichloromethane-methanol-25% conc. ammonia = 60:10:1
L Dichloromethane-methanol-25% conc. ammonia = 90:20:1
M Dichloromethane-methanol-25% conc. ammonia = 200:40:1
N Dichloromethane-methanol-25% conc. ammonia = 200:20:1 + 10% methanol
O Dichloromethane-methanol-25% conc. ammonia = 200:100:2
P Dichloromethane-methanol-25% conc. ammonia = 95:5:1
Q Dichloromethane-methanol-25% conc. ammonia = 200:15:2
R Dichloromethane-methanol-25% conc. ammonia = 200:20:2
S Dichloromethane-methanol-25% conc. ammonia = 200:15:1
T Dichloromethane-methanol-25% conc. ammonia = 200:50:1

### HPLC gradients on Hypersil BDS C-18 (5 µm); column: 4 ×125 mm

I 90% water*/10% acetonitrile* to 0% water*/100% acetonitrile* in 5 minutes + 2.5 minutes (1.5 ml/min)
II 95% water*/5% acetonitrile* to 0% water*/100% acetonitrile* in 40 minutes (0.8 ml/min)
* containing 0.1% trifluoroacetic acid

The following abbreviations are used:
Rf ratio of distance travelled by a substance to separation of the eluent front from the start point in thin-layer chromatography
Rt retention time of a substance in HPLC (in minutes)
m.p. melting point (temperature)

### General method A: (N-BOC deprotection)

The solution of 1 mmol of "N-BOC derivative" in 5 ml of chloroform is admixed successively with 15 ml of methanol and 2.5 ml of 2N HCl and stirred at 60°C over 18 hours. The reaction mixture is cooled to room temperature, poured onto 1M aqueous sodium hydrogencarbonate solution (40 ml) and extracted with tert-butyl methyl ether (2 x 60 ml). The organic phases are washed with brine (1 x 60 ml), dried over sodium sulphate and concentrated by evaporation. The title compound is obtained from the residue by means of flash chromatography (SiO₂ 60F).

### General method B: (N-Cbz deprotection)

The solution of 1 mmol of "N-Cbz derivative" in 15 ml of tetrahydrofuran is hydrogenated in the presence of 100-200 mg of 10% Pd/C at 15-20°C over 2-20 hours. The reaction mixture is clarified by filtration and the filtrate is concentrated by evaporation. The title compound is obtained from the residue by means of flash chromatography (SiO₂ 60F).

### General method C: (9-BBN reduction)

The solution of 1 mmol of "lactam" in 3 ml of tetrahydrofuran is admixed with 9-BBN (0.5M in tetrahydrofuran) (3.2-6.4 equiv.) and stirred at reflux over 1-2 hours (checking of conversion with HPLC). The reaction mixture is cooled to room temperature, admixed with ethanolamine (3.2-6.4 equiv) and concentrated by evaporation. The residue is stirred in 1:1 ethyl acetate-heptane (30 ml) at 0°C overnight and clarified by filtration, and the filtrate is concentrated by evaporation. The title compound is obtained from the residue by means of flash chromatography (SiO₂ 60F).

### General method D: (O-alkylation)

The solution of 1 mmol of "alcohol", 1.0-2.0 mmol of "benzyl halide" in 2.0 ml N,N-dimethylformamide is admixed with stirring at -10°C with 1.1 mmol of sodium hydride dispersion (60%). The reaction mixture is stirred at -10°C over 1 hour and at room temperature over 18 hours. The mixture is poured onto 1 M aqueous sodium hydrogencarbonate solution (50 ml) and extracted with tert-butyl methyl ether (2 x 50 ml). The organic phases are washed successively with water (1 x 50 ml) and brine (1 x 60 ml), dried over sodium sulphate and concentrated by evaporation. The title compound is obtained from the residue by means of flash chromatography (SiO₂ 60F).

### General method E: (chlorination)

The solution of 40 mmol of "benzyl alcohol" in 6.40 ml of pyridine and 100 ml of dichloromethane is slowly added dropwise at 0-5°C to the precooled solution of 7.65 ml of thionyl chloride in 20 ml of dichloromethane. The reaction mixture is stirred at 0°C and then at room temperature for 1 hour each and subsequently poured onto 200 ml of ice-water. The mixture is extracted with dichloromethane (2 x 200 ml). The organic phases are washed successively with 1M aqueous sodium hydrogencarbonate solution (2 x 200 ml) and brine, dried over sodium sulphate and concentrated by evaporation. The title compound is obtained from the residue by means of flash chromatography (SiO₂ 60F).

### General method F: (phenol alkylation I)

The mixture of 20 mmol of "phenol" in 60 ml of N,N-dimethylformamide is stirred with 4.15 g of potassium carbonate and 30 mmol of "halide" or "tosylate" at 100°C over 24 hours. The reaction mixture is then concentrated by evaporation. The residue is admixed with 1 M aqueous sodium hydrogencarbonate solution (40 ml) and extracted with ethyl acetate (2 x 60 ml). The organic phases are washed with brine (1 x 60 ml), dried over sodium sulphate and concentrated by evaporation. The title compound is obtained from the residue by means of flash chromatography (SiO₂ 60F).

### General method G: (phenol alkylation II)

A suspension of 1 mmol of "tosylate", 2 mmol of "phenol", 2 mmol of potassium carbonate and 20 ml of acetonitrile is stirred at 90°C over 24 h. The reaction mixture is then concentrated by evaporation. The residue is then admixed with saturated aqueous sodium hydrogencarbonate solution and extracted with ethyl acetate (2x). The organic phases are washed with brine, dried over sodium sulphate and concentrated by evaporation. The title compound is obtained from the residue by means of flash chromatography (SiO₂ 60F).

### General method H: (tosylation)

A solution of 12 mmol of p-toluenesulphonyl chloride in 15 ml of dichloromethane is added dropwise at 0°C to the solution of 10 mmol of "alcohol", 15 mmol of triethylamine, 1 mmol of 4-dimethylaminopyridine in 90 ml of dichloromethane. The reaction mixture is stirred at room temperature over 2-18 hours. The reaction mixture is diluted with dichloromethane and subsequently washed with water and brine, dried over sodium sulphate and concentrated by evaporation. The title compound is obtained from the residue by means of flash chromatography (SiO₂ 60F).

### General method I: (phenol alkylation III)

A suspension of 1 mmol of "phenol", 1.0-1.5 mmol of "tosylate" or "bromide", 1.5 mmol of caesium carbonate and 2 ml of acetonitrile is stirred at 80°C over 2 hours. The reaction mixture is cooled, poured onto water and extracted with ethyl acetate (2x). The organic phases are washed with brine, dried over sodium sulphate and concentrated by evaporation. The title compound is obtained from the residue by means of flash chromatography (SiO₂ 60F).

### General method J: (alcohol desilylation)

A solution of 1 mmol of "silyl ether" in 5 ml of tetrahydrofuran is admixed with 1.5 - 2.0 mmol of tetrabutylammonium fluoride (1 M solution in tetrahydrofuran) and the solution is stirred at room temperature over 1- 2 hours. The reaction solution is subsequently diluted with water and extracted 2x with tert-butyl methyl ether. The combined organic phases are dried over sodium sulphate and concentrated by evaporation. The title compound is obtained from the residue by means of flash chromatography (SiO₂ 60F).

### General method K: (borane reduction)

The solution of 1 mmol of "lactam" in 3 ml of tetrahydrofuran is admixed with borane-tetrahydrofuran complex (1M in tetrahydrofuran) (3.0 - 6.0 equiv.) and stirred at room temperature over 1-3 hours (monitoring of conversion with HPLC or TLC). The reaction mixture is cooled to room temperature, admixed with methanol (3.0 - 6.0 equiv.) and concentrated by evaporation. The title compound is obtained from the residue by means of flash chromatography (SiO₂ 60F).

### Example 1

### 4-{4-[1-(3-Fluorophenyl)pyrrolidin-3-yloxylphenyl}5-[4-(3-methoxypropyl)-3,4-dihydro-2H-benzo[1,4]oxazin-6-ylmethoxy]piperidin-3-ol

Analogously to method B, 0.373 g of benzyl 4-{4-[1-(3-fluorophenyl)pyrrolidin-3-yloxy]phenyl}-3-hydroxy-5-[4-(3-methoxypropyl)-3,4-dihydro-2h-benzo[1,4]-oxazin-6-ylmethoxy]piperidine-1-carboxylate is used to prepare the title compound.

The starting materials are prepared as follows:

### a) Benzyl 4-{4-[1-(3-fluorophenyl)pyrrolidin-3-yloxy]phenyl}-3-hydroxy-5-[4-(3-methoxypropyl)-3,4-dihydro-2h-benzo[1,4]-oxazin-6-ylmethoxy]piperidine-1-carboxylate

Analogously to method J, 0.590 g of 4-{4-[1-(3-fluorophenyl)pyrrolidin-3-yloxy]phenyl}-3-[4-(3-methoxypropyl)-3,4-dihydro-2H-benzo[1,4]-oxazin-6-ylmethoxy]-5-trisopropylsilanyloxypiperidine-1-carboxylic acid is reacted. The title compound is obtained as a colourless solid. Rf = 0.14 (1:1 EtOAc - heptane); Rt = 5.57.

### b) Benzyl 4-{4-[1-(3-fluorophenyl)pyrrolidin-3-yloxy]phenyl}-3-[4-(3-methoxypropyl)-3,4-dihydro-2H-benzo[1,4]oxazin-6-ylmethoxy]-5-triisopropylsilanyloxypiperidine-1-carboxylate

Analogously to method K, 0.740 g of benzyl 4-{4-[1-(3-fluorophenyl)pyrrolidin-3-yloxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted. The title compound is obtained as a colourless oil. Rf = 0.45 (1:1 EtOAc- heptane).

### c) Benzyl 4-{4-[1-(3-fluorophenyl)pyrrolidin-3-yloxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate

Analogously to method D, 0.852 g of benzyl 4-{4-[1-(3-fluorophenyl)pyrrolidin-3-yloxy]phenyl}-3-hydroxy-5-triisopropylsilanyloxypiperidine-1-carboxylate and 0.507 g of 6-chloromethyl-4-(3-methoxypropyl)-4H-benz[1,4]oxazin-3-one are reacted. The title compound is obtained as a yellowish oil. Rf = 0.45 (1:2 EtOAc- heptane).

### d) Benzyl 4-{4-[1-(3-fluorophenyl)pyrrolidin-3-yloxy]phenyl}-3-hydroxy-5-triisopropylsilanyloxypiperidine-1-carboxylate

Analogously to method I, 0.500 g of benzyl 3-hydroxy-4-(4-hydroxyphenyl)-5-triisopropylsilanyloxypiperidine-1-carboxylate and 0.455 g of 1-(3-fluorophenyl)pyrrolidin-3-yl p-toluene-4-sulphonate are reacted. The title compound is obtained as a colourless oil. Rf = 0.43 (1:1 EtOAc- heptane); Rt = 7.42.

### e) Benzyl 3-hydroxy-4-(4-hydroxyphenyl)-5-triisopropylsilanyloxypiperidin-1-carboxylate

A solution of 3.140 g of 4-(4-hydroxyphenyl)-5-triisopropylsilanyloxypiperidin-3-ol in 90 ml of ethyl acetate is admixed with 90 ml of saturated aqueous sodium hydrogencarbonate solution and 1.57 ml of benzyl chloroformate. The mixture is stirred vigorously for 30 minutes and the phases are then separated. The aqueous phase is extracted with 100 ml of ethyl acetate and the combined organic phases are dried over sodium sulphate and concentrated by evaporation. The title compound is obtained as a colourless solid from the residue by means of flash chromatography (SiO₂ F60). Rf = 0.49 (dichloromethane-methanol-conc. ammonia = 200:20:1); Rt = 5.89.

### f) 4-(4-Hydroxyphenyl)-5-triisoprolsilanyloxypiperidin-3-ol

Analogously to method B, 5.210 g of 4-(4-benzyloxyphenyl)-1-(1-phenylethyl)-5-triisopropylsilanyloxypiperidin-3-ol are reacted. The title compound is obtained as a colourless solid. Rf = 0.19 (dichloromethane-methanol-conc. ammonia = 200:20:1); Rt = 3.80.

### g) 4-(4-Benzyloxyphenyl)-1-(1-phenylethyl)-5-triisopropylsilanyloxypiperidin-3-ol

150 ml of borane-tetrahydrofuran complex (1M in tetrahydrofuran) are added dropwise at 0°C to a solution of 20.00 g of 4-(4-benzyloxyphenyl)-1-(1-phenylethyl)-3-triisopropylsilanyloxy-1,2,3,4-tetrahydropyridine in 280 ml of 1,2-dimethoxyethane. The reaction solution is subsequently stirred at 30 °C over 3 hours. The solution is cooled to room temperature and hydrolysed with 70 ml of water. The hydrolysed solution is stirred for a further 5 minutes and subsequently admixed with 56.00 g of sodium percarbonate, and the suspension is stirred at 50°C over 1 hour. The reaction mixture is poured onto 600 ml of water and extracted with 2 × 500 ml of ethyl acetate. The combined organic phases are washed with 400 ml each of water and brine, and concentrated by evaporation. The title compound is obtained as a yellowish oil from the residue by means of flash chromatography (SiO₂ F60). Rf = 0.23 (1:2 EtOAc- heptane); Rt = 5.75.

### h) 4-(4-Benzyloxyphenyl)-1-(1-phenylethyl)-3-triisopropylsilanyloxy-1,2,3,4-tetrahydropyridine

A suspension of 14.70 g of 4-(4-benzyloxyphenyl)-1-(1-phenylethyl)-1,2,3,4-tetrahydropyridin-3-ol [257928-45-3] in 250 ml of dichloromethane is admixed with 6.80 ml of 2,6-lutidine and cooled to 0°C. 12.60 ml of triisopropysilyl trifluoromethanesulphonate are added dropwise and the mixture is stirred at 0°C for a further 1 hour. The reaction solution is poured onto 400 ml of water and the phases are separated. The aqueous phase is extracted with 200 ml of dichloromethane; the combined organic phases are dried over sodium sulphate and concentrated by evaporation. The title compound is obtained as a yellow-brown oil from the residue by means of flash chromatography (SiO₂ F60) Rf = 0.66 (1:2 EtOAc-heptane); Rt = 5.83.

### i) 1-(3-Fluorophenyl)pyrrolidin-3-yl p-toluene-4-sulphonate

p-Toluenesulphonyl chloride is added in portions to a solution of 0.320 g of 1-(3-fluorophenyl)-pyrrolidin-3-ol, 0.40 ml of triethylamine and 0.022 g of N,N-dimethylaminopyridine in 15 ml of dichloromethane. The solution is left at room temperature over 24 hours and subsequently poured onto 30 ml of saturated aqueous sodium hydrogencarbonate solution. The mixture is extracted with 2 × 50 ml of tert-butyl methyl ether and the combined organic phases are washed with 30 ml of brine, dried over sodium sulphate and concentrated

by evaporation. The title compound is obtained as a light brown solid from the residue by means of flash chromatography (SiO₂ F60). Rf = 0.45 (1:1 EtOAc - heptane); Rt = 5.15.

### j) 1-(3-Fluorophenyl)pyrrolidin-3-ol

A 50 ml Schlenk flask is initially charged with 0.800 g of (R)-(-)-3-pyrrolidinol hydrochloride and 1.350 g of sodium tert-butoxide and admixed under argon with 5 ml of degassed toluene. The suspension is stirred at room temperature over 30 minutes. 1.000 g of 3-fluorobromobenzene is added and the mixture is heated to 90°C. A mixture of 0.272 g of tris(dibenzylidene acetone)dipalladium-chloroform complex and 0.334 g of (±)-2,2'-bis(diphenylphosphino)-1,1'-binaphthyl in 5 ml of degassed toluene is added at 90°C and the mixture is stirred at this temperature over 3 hours. The reaction mixture is filtered through Hyflo and the filtrate is admixed with 5 g of silica gel and concentrated by evaporation. The title compound is obtained as a dark brown oil from the residue by means of flash chromatography (SiO₂ F60). Rf = 0.21 (2:3 EtOAc- heptane); Rt = 3.55.

### k) 6-Chloromethyl-4-(3-methoxypropyl)-4H-benzo[1,4]oxazin-3-one

Analogously to method E, 0.37 g of 6-hydroxymethyl-4-(3-methoxypropyl)-4H-benzo[1,4]oxazin-3-one is reacted. The title compound is obtained as a colourless oil. Rf = 0.60 (2:1 EtOAc - heptane). Rt = 4.05.

### l) 6-Hydroxymethyl-4-(3-methoxypropyl)-4H-benzo[1,4]oxazin-3-one

The suspension of 1.79 g of 6-hydroxymethyl-4H-benzo[1,4]oxazin-3-one, 2.20 ml of 1-chloro-3-methoxypropane, 10g of KF on alumina and 0.033 g of potassium iodide in 150 ml of acetonitrile is stirred at reflux over 72 hours. The mixture is cooled and clarified by filtration, and the filtrate is concentrated by evaporation to dryness. The title compound is obtained from the residue by means of flash chromatography (SiO₂ 60F). Rf = 0.60 (9:1 dichloromethane - methanol); Rt = 2.74.

### m) 6-Hydroxymethyl-4H-benzor[1,4]oxazin-3-one

The mixture of 6.9 g of methyl 3-oxo-3,4-dihydro-2H-benzo[1,4]oxazine-6-carboxylate in 230 ml of tetrahydrofuran is cooled to -40°C. Over 30 minutes, 88.9 ml of diisobutylaluminium hydride (1.5M in toluene) are added dropwise at -40°C. The reaction mixture is stirred at -40°C to -20°C over 1.5 hours and subsequently poured cautiously onto 150 ml of 2N HCl (cold). The organic phase is removed and the water phase is extracted with tetrahydrofuran (5 × 100 ml). The organic phases are washed with brine,(1 × 100 ml), filtered through cotton wool and concentrated by evaporation. The title compound is obtained as beige crystals from the residue by crystallization (from ethanol). Rf = 0.16 (2:1 EtOAc - heptane); Rt = 2.23; m.p.: 186 - 187°C.

According to the process described in Example 1, the following compounds are prepared in an analogous manner:

### Examples

8 4-{4-[3-(3-Fluorophenoxy)propoxy]phenyl}-5-[4-(3-methoxypropyl)-3,4-dihydro-2H-benzo[1,4]oxazin-6-ylmethoxy]piperidin-3-ol
9 5-[4-(3-Methoxypropyl)-3,4-dihydro-2H-benzo[1,4]oxazin-6-ylmethoxyl-4-[4-(3-o-tolyloxypropoxy)phenyl]piperidin-3-ol
12 4-{4-[3-(2-Fluorophenoxy)propoxy]phenyl}-5-[4-(3-methoxypropyl)-3,4-dihydro-2H-benzo[1,4]oxazin-6-ylmethoxy]piperidin-3-ol
16 4-{4-[1-(2-Fluorophenyl)pyrrolidin-3-yloxy]lphenyl}-5-[4-(3-methoxypropyl)-3,4-dihydro-2H-benzo[1,4]oxazin-6-ylmethoxy]peridin-3-ol
17 4-{4-[1-(2,5-Difluorophenyl)pyrrolidin-3-yloxy]phenyl}-5-[4-(3-methoxypropyl)-3,4-dihydro-2H-benzo[1,4]oxazin-6-ylmethoxy]piperidin-3-ol
20 4-{4-[3(R)-(2,5-Difluoro-phenoxy)-butoxy]-phenyl}-5-[4-(3-methoxy-propyl) -3,4-dihydro-2H-benzo[1,4]oxazin-6-ylmethoxy]-piperidin-3-ol

### The starting materials are prepared as follows:

### a) (R)-Toluene-4-sulphonic acid 3-(2,5-difluoro-phenoxy)-butyl ester

According to general procedure H and starting from 0.8 g (R)-3-(2,5-difluoro-phenoxy)-butan-1-ol, the title compound is obtained as a yellowish oil. Rf = 0.45 (EtOAc- heptane 1:2); Rt = 5.10.

### b) (R)-3-(2,5-Difluoro-phenoxy)-butan-1-ol

According to general procedure J and starting from 2 g (R)-[3-(2,5-difluoro-phenoxy)-butoxy]-triisopropyl-silane, the title compound is obtained as a yellowish oil. Rf = 0.25 (EtOAc-Heptan 1:2); Rt = 3.73.

### c) (R)-[3-(2,5-Difluoro-phenoxy)-butoxy]-triisopropyl-silane

According to general procedure I, 1.85 g (S)-methanesulphonic acid 1-methyl-3-triisopropylsilanyloxy-propyl ester are reacted with. 0.859 g 2,5-difluorophenol. The title compound is obtained as a yellowish oil. Rf = 0.26 (EtOAc - heptane 1:5); Rt = 7.09.

### d) (S)-Methanesulphonic acid 1-methyl-3-triisopropylsilanyloxy-propyl ester

Methanesulphonylchloride (0.837 g) is added dropwise to a solution of 1.47 g 4-triisopropylsilanyloxy-butan-2-ol and 0.905 g triethylamine in 40 ml of dry tetrahydrofuran cooled to -15°C. The mixture is stirred for 15 minutes at that temperature then for 2 hours at room temperature. It is then poured into 50 ml ice/water and extracted with tert-butyl methyl ether (2 x100 ml). The combined organic phases are washed with 30 ml 1N HCl and 30 ml brine, dried (sodium sulphate) and evaporated to afford the title compound as a colorless oil. Rf = 0.23 (EtOAc-heptane 1:5).

### e) (S)-4-Triisopropylsilanyloxy-butan-2-ol

Triethylamine (1.173 g) is added dropwise to a solution of 2.246 g triisopropylchlorosilane and 1 g (S)-(+)-1,3-butanediol in 15 ml of dry tetrahydrofuran. The mixture is stirred for 48 hours at room temperature, then is diluted with 400 ml of tert-butyl methyl ether and washed respectively with 30 ml 1N HCl, 50 ml water and 50 ml of brine. The organic phase is dried (sodium sulphate), filtered and evaporated to dryness. The residue is purified by means of flash column chromatography (Si02 60F) to provide the title compound as a colorless oil. Rf = 0.31 (EtOAc - heptane 1:5).

### 23 5-[4-(3Methoxypropyl)-3,4-dihydro-2H-benzo[1,4]oxazin-6-ylmethoxy]-4-{4-[2-(2-methyl-benzyloxy)-ethoxy]-phenyl}-piperidin-3-ol

The starting materials are prepared as follows:

### a) Toluene-4-sulphonic acid 2-(2-methyl-benzyloxy)-ethyl ester

The title compound is obtained as a light yellow oil according to method H starting from 2.900 g 2-(2-methyl-benzyloxy)-ethanol. Rf = 0.32 (EtOAc - heptane 1:2); Rt = 4.99.

### b) 2-(2-Methyl-benzyloxy)-ethanol

Dibutyltin oxide (6.100 g) is added to a solution of ethylene glycol (1.500 g) in toluene (250 ml). The reaction mixture is heated to reflux for 24 hours in a Dean-Stark apparatus. The reaction mixture is cooled to 90°C and tetrabutylammonium bromide (1.550 g) and 2-methylbenzyl bromide (6.62 ml) are added. Ca. 50 ml of toluene are distilled off and the remaining reaction mixture is then heated to reflux for 2 hours. The reaction mxture is concentrated under reduced pressure and the residue purified by means of flash column chromatography (SiO₂ 60F) to provide the title compound as a light yellow oil. Rf = 0.25 (EtOAc - heptane 1:1); Rt = 3.20.

### Example 2

4-{4-[3-(2-Methoxybenzyloxy)propoxy]phenyl}-5-[4-(3-methoxypropyl)-3,4-dihydro-2H-benzo[1,4]oxazin-6-ylmethoxy]piperidin-3-ol Analogously to method B, 1.000 g of benzyl 3-hydroxy-4-{4-[3-(2-methoxybenzyloxy)-propoxy]phenyl}-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:

### a) Benzyl 3-hydroxy-4-{4-[3-(2-methoxybenzyloxy)propoxy]phenyl}-5-[4-(3-methoxypropyl)-3,4-dihydro-2H-benzo[1,4]oxazin-6-ylmethoxy]piperidine-1-carboxylate

Analogously to method J, 3.100 g of benzyl 4-{4-[3-(2-methoxybenzyloxy)propoxy]phenyl}-5-[4-(3-methoxypropyl)-3,4-dihydro-2H-benzo[1,4]oxazin-6-ylmethoxy]-5-triisopropylsilanyloxypiperidine-1-carboxylate are reacted. The title compound is obtained as a yellow resin.

Rf = 0.25 (3:1 EtOAc - heptane); Rt = 5.50.

### b) Benzyl 4-{4-[3-(2-methoxybenzyloxy)propoxy]phenyl}-5-[4-(3-methoxypropyl)-3,4-dihydro-2H-benzo[1,4]oxazin-6-ylmethoxy]-5-triisopropylsilanyloxypiperidine-1-carboxylate

Analogously to method C, 3.800 g of benzyl 4-{4-[3-(2-methoxybenzyloxy)propoxy]phenyl}-5-[4-(3-methoxypropyl)-3-oxo-3,4-dihydro-2H-benzo[1,4]oxazin-6-ylmethoxy]-5-triisopropylsilanyloxypiperidine-1-carboxylate are reacted. The title compound is obtained as a yellowish oil. Rf = 0.33 (1:1 EtOAc - heptane).

### c) Benzyl 4-{4-[3-(2-methoxybenzyloxy)propoxy]pheny}-5-[4-(3-methoxypropyl)-3-oxo-3,4-dihydro-2H-benzo[1,4]oxazin-6-ylmethoxy]-5-tiisopropylsilanyloxypiperidine-1-carboxylate

Analogously to method D, 3.150 g of benzyl 3-hydroxy-4-{4-[3-(2-methoxybenzyioxy)-propoxy]phenyl}-5-triisopropylsilanyloxypiperidine-1-carboxylate and 1.583 g of 6-chloromethyl-4-(3-methoxypropyl)-4H-benzo[1,4]oxazin-3-one (Example 1k) are reacted. The title compound is obtained as a yellowish oil. Rf = 0.33 (1:1 EtOAc - heptane); Rt = 7.64.

### d) Benzyl 3-hydroxy-4-{4-[3-(2-methoxybenzyloxy)propoxy]phenyl}-5-triisopropylsilanyloxypiperidine-1-carboxylate

Analogously to method I, 2.500 g of Benzyl 3-hydroxy-4-(4-hydroxyphenyl)-5-triisopropylsilanyloxypiperidine-1-carboxylate (Example 1 e) and 2.017 g of 3-(2-methoxybenzyloxy)propyl p-toluene-4-sulphonate are reacted. The title compound is obtained as a yellow oil. Rf = 0.43 (1:1 EtOAc - heptane); Rt = 7.42.

### e) 3-(2-Methoxybenzyloxy)propyl p-toluene-4-sulphonate

Analogously to method J, 59.00 g of 3-(2-methoxybenzyloxy)propan-1-ol [188879-03-0] are reacted. The title compound is obtained as a colourless solid. Rf = 0.21 (1:4 EtOAc-heptane); Rt = 5.05.

### Example 3

### 4-(4-Methoxyphenyl)-5-[4-(3-methoxypropyl)-3,4-dihydro-2H-benzo[1,4]oxazin-6-ylmethoxy]piperidin-3-ol

Analogously to method B, 0.0352 g of benzyl 3-hydroxy-4-(4-methoxyphenyl)-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:

### a) Benzyl 3-hydroxy-4-(4-methoxyphenyl)-5-[4-(3-methoxypropyl)-3,4-dihydro-2H-benzo[1,4]oxazin-6-ylmethoxy]piperidine-1-carboxylate

Analogously to method J, 0.170 g of benzyl 4-(4-methoxyphenyl)-3-[4-(3-methoxypropyl)-3,4-dihydro-2H-benzo[1,4]oxazin-6-ylmethoxy]-5-triisopropylsilanyloxypiperidine-1-carboxylate is reacted. The title compound is obtained as a whitish oil. Rf = 0.05 (1:1 EtOAc - heptane); Rt = 4.72.

### b) Benzyl 4-(4-methoxyphenyl)-3-[4-(3-methoxypropyl)-3,4-dihydro-2H-benzo[1,4]oxazin-6-ylmethoxy]-5-triisopropylsilanyloxypiperidine-1-carboxylate

Analogously to method K, 0.200 g of benzyl 4-(4-methoxyphenyl)-3-[4-(3-methoxypropyl)-3-oxo-3,4-dihydro-2H-benzo[1,4]oxazin-6-ylmethoxy]-5-tiisopropylsilanyloxypiperidine-1-carboxylate is reacted at 60°C. The title compound is obtained as a colourless oil. Rf = 0.50 (1:1 EtOAc -heptane); Rt = 5.10.

### c) Benzyl 4-(4-methoxyphenyl)-3-[4-(3-methoxypropyl)-3-oxo-3,4-dihydro-2H-benzo[1,4]oxazin-6-ylmethoxy]-5-triisopropylsilanyloxypiperidine-1-carboxylate

Analogously to Example 1c, 0.375 g of benzyl 3-hydroxy-4-(4-methoxyphenyl)-5-triisopropylsilanyloxypiperidine-1-carboxylate and 0.210 g of 6-chloromethyl-4-(3-methoxypropyl)-4H-benzo[1,4]oxazin-3-one are reacted. The title compound is obtained as a colourless oil. Rf = 0.46 (1:1 EtOAc - heptane); Rt = 7.02.

### d) Benzyl 3-hydroxy-4-(4-methoxyphenyl)-5-triisopropylsilanyloxypiperidine-1-carboxylate

A solution of 0.440 g of benzyl 3-hydroxy-4-(4-hydroxyphenyl)-5-triisopropylsilanyloxypiperidine-1-carboxylate (Example 1e) in 6 ml of acetone is admixed with 0.088 ml of dimethyl sulphate and 0.163 g of potassium carbonate. The suspension is stirred at 65°C for 18 hours. After cooling, the reaction mixture is flitered and concentrated by evaporation. The residue is admixed with 30 ml of water and extracted with 100 ml of tert-butyl methyl ether. The organic phase is washed with 30 ml of brine, dried over sodium sulphate and concentrated by evaporation. The tittle compound is obtained as a colourless oil from the residue by means of flash chromatography (SiO₂ F60). Rt = 6.47.

### Example 4

### 6-[5-Methoxy-4-(4-methoxyphenyl)piperidin-3-yloxymethyl]-4-(3-methoxypropyl)-3,4-dihydro-2H-benzo[1,4]oxazine

Analogously to method B, 0.0672 g of benzyl 3-methoxy-4-(4-methoxyphenyl)-5-[4-(3-methoxypropyl)-3,4-dihydro-2H-benzo[1,4]oxazin-6-ylmethoxy]piperidine-1-carboxylate is used to prepare the title compound.

The starting material is prepared as follows:

### a) Benzyl 3-methoxy-4-(4-methoxyphenyl)-5-[4-(3-methoxypropyl)-3,4-dihydro-2H-benzo[1,4]oxazin-6-ylmethoxy]piperidine-1-carboxylate

Analogously to method D, 0.0758 g of benzyl 3-hydroxy-4-(4-methoxyphenyl)-5-[4-(3-methoxypropyl)-3,4-dihydro-2H-benzo[1,4]oxazin-6-ylmethoxy]piperidine-1-carboxylate (Example 3a) was reacted. The title compound is obtained as a colourless oil. Rt = 5.29.

### Example 5

### 6-(5-Methoxy-4-{4-[3-(2-methoxybenzyloxy)propoxy]phenyl}piperidin-3-yloxymethyl)-4-(3-methoxypropyl)-3,4-dihydro-2H-benzo[1,4]oxazine

Analogously to method B, 0.145 g of benzyl 3-methoxy-4-{4-[3-(2-methoxybenzyloxy)propoxy]phenyl}-5-[4-(3-methoxypropyl)-3,4-dihydro-2H-benzo[1,4]-oxazin-6-ylmethoxy]piperidine-1-carboxylate is used to prepare the title compound.

The starting materials are prepared as follows:

### a) Benzyl 3-methoxy-4-{4-[3-(2-methoxybenzyloxy)propoxy]phenyl}-5-[4-(3-methoxypropyl)-3,4-dihydro-2H-benzo[1,4]oxazin-6-ylmethoxy]piperidine-1-carboxylate

A solution of 0.276 g of benzyl 3-hydroxy-4-{4-[3-(2-methoxybenzyloxy)propoxy]phenyl}-5-[4-(3-methoxypropyl)-3,4-dihydro-2H-benzo[1,4]oxazin-6-ylmethoxy]piperidine-1-carboxylate (Example 2a) and 0.070 ml of methyl iodide in 5.0 ml of N,N-dimethylformamide is admixed with stirring at -10°C with 0.019 g of sodium hydride dispersion (60%). The reaction mixture is stirred at -10°C over 1 hour and at room temperature for 18 hours. The mixture is poured onto 1 M aqueous sodium hydrogencarbonate solution (50 ml) and extract with tert-butyl methyl ether (2 × 50 ml). The organic phases are washed successively with water (1 × 50 ml) and brine (1 × 60 ml), dried over sodium sulphate and concentrated by evaporation. The title compound is obtained as a colourless oil from the residue by means of flash chromatography (SiO₂ 60F). Rf = 0.37 (2:1 EtOAc - heptane); Rt = 5.92.

### Example 6

### 4-{4-[3-(2-Chlorophenoxy)propoxy]phenyl}-5-[4-(3-methoxypropyl)-3,4-dihydro-2H-benzo[1,4]oxazin-6-ylmethoxy]piperidin-3-ol

0.125 g of benzyl 4-{4-[3-(2-chlorophenoxy)propoxy]phenyl}-3-hydroxy-5-[4-(3-methoxypropyl)-3,4-dihydro-2H-benzo[1,4]oxazin-6-ylmethoxy]piperidine-1-carboxylate are dissolved in 3.8 ml of dioxane. 3.8 ml each of methanol and 40% aqueous potassium hydroxide solution are added and the reaction mixture is stirred in a sealed flask over 14 hours. The reaction mixture is subsequently cooled to room temperature, diluted with 30 ml of water and extracted with ethyl acetate (3 × 60 ml). The combined organic phases are washed with 50 ml of water, dried (sodium sulphate), filtered and concentrated by evaporation. The title compound is obtained from the residue by means of flash chromatography (SiO₂ 60F).

The starting materials are prepared as follows:

### a) Benzyl 4-{4-[3-(2-chlorophenoxy)propoxy]phenyl}-3-hydroxy-5-[4-(3-methoxypropyl)-3,4-dihydro-2H-benzo[1,4]oxazin-6-ylmethoxy]piperidine-1-carboxylate

Analogously to method J, 0.386 g of benzyl 4-{4-[3-(2-chlorophenoxy)propoxy]phenyl}-3-[4-(3-methoxypropyl)-3,4-dihydro-2H-benzo[1,4]oxazin-6-ylmethoxy]-5-triisopropylsilanytoxypiperidine-1-carboxylate is reacted. The title compound is obtained as a yellowish oil.

Rf = 0.16 (1:1 EtOAc/heptane) ; Rt = 5.64.

### b) Benzyl 4-{4-[3-(2-chlorophenoxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate

Analogously to method K, 0.525 g of benzyl 4-{4-[3-(2-chlorophenoxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted at 60°C. The title compound is obtained as a orange oil. Rf = 0.08 (1:3 EtOAc/heptane).

### c) Benzyl 4-{4-[3-(2-chlorophenoxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate

Analogously to Example 1c, 0.460 g of benzyl 4-{4-[3-(2-chlorophenoxy)propoxy]phenyl}-3-hydroxy-5-triisopropylsilanyloxypiperidine-1-carboxylate and 0.213 g of 6-chloromethyl-4-(3-methoxypropyl)-4H-benzo[1,4]oxazin-3-one are reacted. The title compound is obtained as an orange oil. Rf = 0.10 (1:3 EtOAc-heptane); Rt = 7.68.

### d) Benzyl 4-{4-[3-(2-chlorophenoxy)propoxy]phenyl}-3-hydroxy-5-triisopropylsilanyloxypiperidine-1-carboxylate

Analogously to method I, 0.400 g of benzyl 3-hydroxy-4-(4-hydroxyphenyl)-5-triisopropylsilanyloxypiperidine-1-carboxylate (Example 1e) and 0.241 g of 1-(3-bromopropoxy)-2-chlorobenzene [50912-59-9] are reacted. The title compound is obtained as an orange oil. Rf = 0.10 (1:3 EtOAo-heptane); Rt = 7.00.

### Example 7

### 6-(4-{4-[1-(3-Fluorophenyl)pyrrolidin-3-yloxy]phenyl}-5-methoxypiperidin-3-yloxymethyl-4-(3-methoxypropyl)-3,4-dihydro-2H-benzo[1,4]oxazine

Analogously to Example 5, benzyl 4-{4-[1-(3-fluorophenyl)pyrrolidin-3-yloxy]phenyl}-3-hydroxy-5-[4-(3-methoxypropyl)-3,4-dihydro-2H-benzo[1,4]oxazin-6-ylmethoxy]piperidine-1-carboxylate is used to prepare the title compound.

### Example 10

### 4-{4-[3-(2-Fluorobenzyloxy)propoxy]phenyl}-5-[4-(3-methoxypropyl)-3,4-dihydro-2H-benzo[1,4]oxazin-6-ylmethoxy]piperidin-3-ol

Analogously to method B, 0.144 g of benzyl 4-{4-[3-(2-fluorobenzyloxy)propoxy]phenyl}-3-hydroxy-5-[4-(3-methoxypropyl)-3,4-dihydro-2H-benzo[1,4]oxazin-6-ylmethoxy]piperidine-1-carboxylate is used to prepare the tithe compound.

The starting materials are prepared as follows:

### a) Benzyl 4-{4-[3-(2-fluorobenzyloxy)propoxy]phenyl}-3-hydroxy-5-[4-(3-methoxypropyl)-3,4-dihydro-2H-benzo[1,4]oxazin-6-ylmethoxy]piperidine-1-carboxylate

Analogously to method J, 0.522 g of benzyl 4-{4-[3-(2-fluorobenzyloxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate is reacted. The title compound is obtained as a whitish oil. Rf = 0.15 (1:1 EtOAc-heptane); Rt = 5.51.

### b) Benzyl 4-{4-[3-(2-fluorobenzyloxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate

Analogously to method K, 0.688 g of benzyl 4-{4-[3-(2-fluorobenzyloxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted at 60°C. The title compound is obtained as a colourless oil. Rf = 0.49 (1:1 EtOAclheptane).

### c) Benzyl 4-{4-[3-(2-fluorobenzyloxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate

Analogously to Example 1c, 0.548 g of benzyl 4-{4-[3-(2-fluorobenzyloxy)propoxy]phenyl}-3-hydroxy-5-triisopropylsilanyloxypiperidine-1-carboxylate and 0.236 g of 6-chloromethyl-4-(3-methoxypropyl)-4H-benzo[1,4]oxazin-3-one are reacted. The title compound is obtained as a yellowish oil. Rt = 7.57.

### d) Benzyl 4-{4-[3-(2-fluorobenzyloxy)propoxy]phenyl}-3-hydroxy-5-triisopropylsilanyloxypiperidine-1-carboxylate

Analogously to method I, 0.400 g of benzyl 3-hydroxy-4-(4-hydroxyphenyl)-5-triisopropylsilanyloxypiperidine-1-carboxylate (Example 1e) and 0.298 g of 3-(2-fluorobenzyloxy)propyl p-toluene-4-sulphonate. The title compound is obtained as a yellowish oil. Rf = 0.54 (1:1 EtOAc-heptane); Rt = 6.89.

e) 3-(2-Fluorobenzyxy)propyl p-toluene-4-sulphonate

Analogously to method H, 5.94 g of 3-(2-fluorobenzyloxy)propan-1-ol are reacted. The title compound is obtained as a white solid. Rf = 0.27 (1:4 EtOAo-heptane) ; Rt = 4.99.

f) 3-(2-Fluorobenzyloxy)propan-1-ol

A solution of 10 g 2-fluorobenzaldehyde, 7.21 g of propanediol and 0.20 g of pyridinium p-tolunesulphonate in 120 ml of hexane is stirred at 90°C in a water separator for 17 hours and then concentrated. The residue is dissolved in 90 ml of toluene and cooled at 0°C. 150 ml of diisobutylaluminium hydride (25% in toluene) are slowly added dropwise and the reaction mixture is stirred at 0°C for 2 hours. A solution of 33 g of citric acid monohydrate in 120 ml of water is added dropwise, followed by 120 ml of 2N HCl. The mixture is stirred at 0°C for another 1 hour, poured onto 150 ml of water and extracted twice with toluene. The combined organic phases are washed with 1M sodium hydrogencarbonate solution and brine, dried over sodium sulphate, filtered and concentrated by evaporation. The title compound is obtained as a colourless oil from the residue by means of flash chromatography (SiO₂ F60). Rf = 0.39 (2:1 EtOAc-heptane); Rt = 3.22.

### Example 11

### 4-{4-[3-(2,5-Difluorophenoxy)propoxy]phenyl}-5-[4-(3-methoxypropyl)-3,4-dihydro-2H-benzo[1,4]oxazin-6-ylmethoxy]piperidin-3-ol

Analogously to method B, 0.140 g of benzyl 4-{4-[3-(2,5-difluorophenoxy)propoxy]phenyl}-3-hydroxy-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:

### a) Benzyl 4-{4-[3-(2,5-difluorophenoxy)propoxy]phenyl}-3-hydroxy-5-[4-(3-methoxypropyl)-3,4-dihydro-2H-benzo[1,4]oxazin-6-ylmethoxy]piperidine-1-carboxylate

Analogously to method J, 0.421 g of benzyl 4-{4-[3-(2,5-difluorophenoxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate is reacted. The title compound is obtained as a whitish oil. Rf = 0.15 (1:1 EtOAc-heptane); Rt = 5.47.

### b) Benzyl 4-{4-[3(-(2,5-difluorophenoxy)propoxy]phenyl}-3-[4-(3-methoxypropyl)-3,4-dihydro-2H-benzo[1,4]oxazin-6-ylmethoxy]-5-triisopropylsilanyloxypiperidine-1-carboxylate

Analogously to method K, 0.620 g of benzyl 4-{4-[3-(2,5-difluorophenoxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted at 60°C. The title compound is obtained as a colourless oil. Rf = 0.47 (1:1 EtOAo-heptane).

### c) Benzyl 4-{4-[3-(2,5-difluorophenoxy)propoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate

Analogously to Example 1c, 0.453 g of benzyl 4-{4-[3-(2,5-difluorophonoxy)propoxy]phenyl}-3-hydroxy-5-triisopropylsilanyloxypiperidine-1-carboxylate and 0.209 g of 6-chloromethyl-4-(3-methoxypropyl)-4H-benzo[1,4]oxazin-3-one are reacted. The title compound is obtained as a yellow oil. Rf = 0.47 (1:1 EtOAo-heptane). Rt = 7.26.

### d) Benzyl 4-{3-(2,5-difluorophenoxy)propoxy]phenyl}-3-hydroxy-5-triisopropylsilanyloxypiperidine-1-carboxylate

Analogously to method I, 0.400 g of benzyl 3-hydroxy-4-(4-hydroxyphenyl)-5-triisopropylsilanyloxypiperidine-1-carboxylate (Example 1e) and 0.236 g of 2-(3-bromopropoxy)-1,4-difluorobenzene are reacted. The title compound is obtained as a colourless oil. Rf = 0.09 (1:3 EtOAc-heptane). Rt = 6.74.

### e) 2-(3-Bromopropoxy)-1,4-difluorobenzene

The mixture of 9.78 g of 2,5-difluorophenol, 150.24 g of 1,3-dibromopropane and 15.585 g of potassium carbonate in 160 ml of acetonitrile is stirred at reflux over 16 hours. The reaction mixture is cooled, diluted with 400 ml of water and extracted twice with 400 ml of tert butyl methyl ether. The combined organic phases are washed with 400 ml of 1N NaOH and 300 ml of brine, dried over sodium sulphate, filtered and concentrated by evaporation. The title compound is obtained as a colourless oil from the residue by means of flash chromatography (SiO₂ 60F). Rf = 0.66 (1:1 EtOAc-heptane); Rt = 4.89.

### Example 13

### 4-{4-[2-(3-Fluorobenzyloxy)ethoxy]pheny}-5-[4-(3-methoxypropyl)-3,4-dihydro-2H-benzo[1,4]oxazin-6-ylmethoxy]piperidin-3-ol

Analogously to method B, 0.200 g of benzyl 4-{4-[2-(3-fluorobenzyloxy)ethoxy]phenyl}-3-hydroxy-5-[4-(3-methoxypropyl)-3,4-dihydro-2H-benzo[1,4]oxazin-6-ylmethoxy]piperidine-1-carboxylate is used to prepare the title compound.

The starting materials are prepared as follows:

### a) Benzyl 4-{4-[2-(3-fluorobenzyloxy)ethoxy]phenyl}-3-hydroxy-5-[4-(3-methoxypropyl)-3,4-dihydro-2H-benzo[1,4]oxazin-6-ylmethoxy]piperidine-1-carboxylate

Analogously to method J, 0.410 g of benzyl 4-{4-[2-(3-fluorobenzyloxy)ethoxy]phenyl}-3-[4-(3-methoxypropyl)-3,4-dihydro-2H-benzo[1,4]oxazin-6-ylmethoxy]triisopropylsilanyloxypiperidine-1-carboxylate is reacted. The tittle compound is obtained as a yellowish resin.

Rf = 0.13 (2:1 EtOAc - heptane); Rt = 5.31.

### b) Benzyl 4-{4-[2-(3-fluorobenzyloxy)ethoxy]phenyl}-3-[4-(3-methoxypropyl)-3,4-dihydro-2H-benzo[1,4]oxazin-6-ylmethoxy]triisopropylsilanyloxypiperidine-1-carboxylate

Analogously to method K, 0.590 g of butyl 4-{4-[2-(3-fluorobenzyloxy)ethoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate is reacted. The title compound is obtained as a yellowish oil. Rf = 0.40 (1:1 EtOAc - heptane).

### c) Benzyl 4-{4-[2-(3-fluorobenzyloxy)ethoxy]phenyl}-3-[4-(3-methoxypropyl)-3-oxo-3,4-dihydro-2H-benzo[1,4]oxazin-6-ylmethoxy]-5-triisopropylsilanyloxypiperidine-1-carboxylate

Analogously to method D, 0.575 g of benzyl 4-{4-[2-(3-fluorobenzyloxy)ethoxy]phenyl}-3-hydroxy-5-triisopropylsilanyloxypiperidine-1-carboxylate and 0.300 g 6-chloromethyl-4-(3-methoxypropyl)-4H-benz[1,4]oxazin-3-one (Example 1k). The title compound is obtained as a bright yellow oil. Rf = 0.29 (1:1 EtOAc- heptane); Rt = 7.18.

### d) Benzyl 4-{4-[2-(3-fluorobenzyloxy)ethoxy]phenyl}-3-hydroxy-5-triisopropylsilanyloxypiperidine-1-carboxylate

Analogously to method 1, 0.500 g of benzyl 3-hydroxy-4-(4-hydroxyphenyl)-5-triisopropylsilanyloxypiperidine-1-carboxylate (Example 1e) and 2.017 g of 2-(3-fluorobenzyloxy)ethyl p-toluene-4-sulphonate are reacted. The title compound is obtained as a yellow oil. Rf = 0.43 (1:1 EtOAc- heptane); Rt = 7.42.

### e) 2-(3-Fluorobenzyloxy)ethyl p-toluene-4-sulphonate

Analogously to method J, 3.010 g of 2-(3-fluorobenzyloxy)ethanol are reacted. The title compound is obtained as a yellowish oil. Rf = 0.32 (1:2 EtOAc - heptane); Rt = 4.82.

### f) 2-(3-Fluorobenzyloxy)ethanol

A solution of 1.47 ml of ethylene glycol in 360 ml of toluene is admixed with 6.650 g of dibutyltin oxide and the reaction solution is subsequently heated to reflux on a water separator over 20 hours. The reaction solution is cooled gently and admixed with 3.380 g of tetrabutylammonium bromide and 10.00 g of 3-fluorobenzyl bromide. 50 ml of toluene are distilled off and the reaction mixture is subsequently heated at reflux over 2 hours. The reaction mixture is concentrated by evaporation and the title compound is obtained as a yellowish liquid from the residue by means of flash chromatography (SiO₂ 60F). Rf = 0.23 (1:1 EtOAc - heptane); Rt = 3.04.

According to the process described in Example 13, the following compounds are prepared in an analogous manner:

### Examples

14 4-{4-[2-(2,5-Difluorobenzyloxy)ethoxy]phenyl}-5-[4-(3-methoxypropyl)-3,4-dihydro-2H-benzo[1,4]oxazin-6-ylmethoxy]piperidin-3-ol
15 4-{4-[2-(2-Fluorobenzyloxy)ethoxy]phenyl}-5-[4-(3-methoxypropyl)-3,4-dihydro-2H-benzo[1,4]oxazin-6-ylmethoxy]piperidin-3-ol
24 4-{4-[3-(Adamantan-1-yloxy)-propoxy]-phenyl}-5-[4-(3-methoxy-propyl)-3,4-dihydro-2H-benzo[1,4]oxazin-6-ylmethoxy]-piperidin-3-ol

The starting materials are prepared as follows:

### a) Toluene-4-sulphonic acid 3-(adamantan-1-yloxy)-propyl ester

The solution of 0.941 g of 3-(adamantan-1-yloxy)-propan-1-ol in 12 ml of dichloromethane is admixed with 0.99 ml triethylamine, 0.061 g 4-dimethylaminopyridine, 1.07 g p-toluenesulphonyl chloride and then stirred for 3 hours at room temperature. The reaction mixture is subsequently poured onto 50 ml of water and extracted twice with 50 ml of dichloromethane. The combined organic phases are washed with 50 ml of brine, dried over sodium sulphate, filtered and concentrated by evaporation. The title compound is obtained as a colourless oil from the residue by means of flash chromatography (SiO₂ 60F). Rf = 0.33 (1:3 EtOAc-heptane); Rt = 5.91.

### b) 3-(Adamantan-1-yloxy)-propan-1-ol

The stirred solution of 1.25 g 1-allyloxy-adamantane in 85 ml of tetrahydrofuran is treated with 1.62 ml borane-methylsulfide comlex at 0°C. The reaction solution is heated to reflux over 1.5 hours, subsequently cooled to 5°C and admixed with 1.61 ml of 2M NaOH and 0.33 ml hydrogen peroxide (30%). The mixture is heated to reflux and stirred for an additional 1.5 hours, subsequently cooled to room temperature and admixed with 85 ml of 1M potassium carbonate solution. The mixture is extracted with tert-butyl methyl ether (2 × 80 ml) and the combined organic phases are washed with 40 ml of brine, dried over sodium sulphate and concentrated by evaporation. The title compound is obtained as a colorless oil from the residue by means of flash chromatography (SiO₂ 60F). Rf = 0.38 (1:1 EtOAc-heptane).

### c) 1-Allyloxy adamantane

A mixture of 0.434 g sodium hydride and 75 ml of anhydrous tetrahydrofuran is admixed with 1.0 g 1-adamantol and 0.936 ml allylbromide. The reaction mixture is heated to reflux and stirred for additional 18 hours. The reaction mixture is cooled to room temperature, poured onto 150 ml of water and extracted with tert-butyl methyl ether (2 × 150 ml). The combined organic phases are washed with 100 ml of brine, dried over sodium sulphate and concentrated by evaporation The title compound is obtained as a yellow oil from the residue by means of flash chromatography (SiO₂ 60F). Rf = 0.79 (1:2 EtOAc-heptane).
25 5-[4-(3-Methoxy-propyl)-3,4-dihydro-2H-benzo[1,4]oxazin-6-ylmethoxy]-4-{4-[3-(1-methyl-cyclohexyloxy)-propoxy]-phenyl]-piperidin-3-ol

### Example 18

### 6-[5-(2-Methoxyethoxy)-4-(4-methoxyphenyl)piperidin-3-yloxymethyl]-4-(3-methoxypropyl)-3,4.djhydro-2H-benzo[1,4]oxazine

Analogously to method B, 0.027 g of benzyl 3-(2-methoxyethoxy)-4-(4-methoxyphenyl)-5-[4-(3-methoxypropyl)-3,4dihydro-2H-benzo[1,4]oxazin-6-ylmethoxy]piperidine-1-carboxylate is used to prepare the title compound.

The starting materials are prepared as follows:

### a) Benzyl 3-(2-methoxyethoxy)4-(4-methoxyphenyl)-5-[4-(3-methoxypropyl)-3,4dihydro-2H-benzo[1,4]oxazin-6-ylmethoxy]piperidine-1-carboxylate

A solution of 0.156 g of benzyl 3-hydroxy-4-(4-methoxyphenyl)-5-[4-(3-methoxypropyl)-3,4,dihydro-2H-benzo[1,4]oxazin-6-ylmethoxy]piperidine-1-carboxylate (Example 3a) in 4 ml of anhydrous tetrahydrofuran is admixed with 0.028 g of sodium hydride (60% dispersion in paraffin). The reaction mixture is stirred at room temperature for 10 minutes and subsequently heated to reflux. A solution of 0.083 g of 2-methoxyethyl p-toluenesulphonate in 2 ml of tetrahydrofuran is added dropwise over 5 minutes and the reaction mixture is subsequently heated to reflux over 16 hours. The reaction mixture is cooled, admixed with 10 ml of water and extracted with tert-butyl methyl ether (2 × 20 ml). The combined organic phases are washed with 20 ml of brine, dried over sodium sulphate and concentrated by evaporation. The title compound is obtained as a colourless oil from the residue by means of flash chromatography (SiO₂ 60F). Rf = 0.30 (2:1 EtOAc - heptane); Rt = 5.21.

### Example 19

### 6-[5-Cyclohexyloxy-4(4-methoxy-phenyl)-piperidin-3-yloxymethyl]-4-(3-methoxy-propyl)-3,4-dihydro-2H-benzo[1,4]oxazine

The title compound is prepared according to method C starting from 0.039 g of 3-(cyclohex 2-enyloxy)4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:

### a) 3-(Cyclohex-2-enyloxy)-4-(4-methoxy-phenyl)-5-[4-(3-methoxy-propyl)-3,4-dihydro-2H-benzo[1,4]oxazin-6-ylmethoxy]l-piperidine-1-carboxylic acid benzyl ester

According to method D, 0.105 g of 3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (example 3a) are reacted with 0.065 g of 3-bromo cyclohexene. The title compound is obtained as a colorless oil. Rf = 0.45 (EtOAc- heptane 2:1); Rt = 5.85.

### Example 21

### 6-[5-Cyclopentyloxy-4(4-methoxy-phenyl)-piperidin-3-yloxymethyl]-4-(3-methoxy-propyl)-3,4-dihydro-2H-benzo[1,4]oxazine

The title compound was prepared according to method C starting from 0.035 g of 3-(cyclopent-2-enyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:

### a) 3-(Cyclopent-2-enyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester

A solution of 0.105g 3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (example 3a) and 0.061 g 2,2,2-trichloro-acetimidic acid cyclopent-2-enyl ester (CAS 748780-85-0) in 6 ml of dichloromethane is cooled to -60°C under argon. Boron trifluoro ethyletherate (0.023 ml) is added dropwise and the reaction mixture is stirred at -60°C to -40°C for 2 hours. The reaction mixture is quenched with saturated aqueous sodium bicarbonate solution and is extracted with dichloromethane (2x). The combined organics are washed with brine, dried (sodium sulphate) and concentrated under reduced pressure. The residue is purified by means of flash column chromatography (SiO₂ 60F) to provide the title compound as a colorless oil. Rf = 0.50 (EtOAc - heptane 2:1); Rt = 5.69.

### Example 22

### 4-{4-[2-(2,5-Difluoro-phenoxy)-ethoxymethyl]-phenyl}-5-[4-(3-methoxy-proyl)-3,4-dihydro-2H-benzo[1,4]oxazin-6-ylmethoxy]-piperidin-3-ol

The title compound is prepared according to method B starting from 0.08 g of 4-{4-[2-(2,5-difluoro-phenoxy)-ethoxymethyl]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester.

The starting materials are prepared as follows:

### a) 4-{2-[2,5-Difluoro-phenoxy)-ethoxymethyl]-phenyl}-3-hydroxy-5-[4-(3-methoxy-propyl)-3,4-dihydro-2H-benzo[1,4]oxazin-6ylmethoxy]-piperidine-1-carboxylic acid benzyl ester

According to method J, 0.241 g 4-{4-[2-(2,5-difluoro-phenoxy)-ethoxymethyl]-phenyl}-3-[4-(3-methoxy-propyl)-3,4-dihydro-2H-benzo[1,4]oxazin-6-ylmethoxy]-5-triisopropylsilanyloxypiperidine-1-carboxylic acid benzyl ester are reacted to afford the title compound as a colorless oil. Rf = 0.38 (EtOAc - heptane 2:1). Rt = 5.26.

### b) 4{4-[2-(2,5-Difluoro-phenoxy)-ethoxymethyl]-phenyl}-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester

According to method D, 0.458 g 4-(4-chloromethyl-phenyl)-3-[4-(3-methoxy-propyl)3,4-dihydro-2H-benzo[1,4]oxazin-6-ylmethoxy]-5-triisopropylsilanyloxy-piperidine-1-carboxylic acid benzyl ester are reacted with 0.159 g 2-(2,5-difluoro-phenoxy)-ethanol. The title compound is obtained as a colorless oil. Rf = 0.45 (EtOAc - heptane 1:2).

### c) 4-(4-Chloromethyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester

2.82 g of chlorenamine are added dropwise to a solution of 1 g 4-(4-hydroxymethyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester in 20 ml of dry methylene chloride cooled to 0°C. The reaction mixture is stirred for 5 minutes at 0°C and for 18 hours at room temperature. It is then poured into 100 ml water and 100 ml tert-butylmethylether. The mixture is vigorously stirred for 1 hour at room temperature. The organic phase is separated, washed with 75 ml brine, dried over sodium sulphate and evaporated under reduced pressure. The residue is purified by means of flash column chromatography (Si02 60F) to provide the title compound as a yellow oil. Rf = 0.40 (EtOAc- heptane 1:1.5).

### d) 4-(4-Hydroxymethyl-pheny)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester

According to method C, 6.25 g 4-(4-methoxycarbonyl-phenyl)-3-[4-(3-methoxy-propyl)-3-oxo-3,4-dihydro-2H-benzo[1,4]oxazin-6-ylmethoxy]-5-triisopropylsilanyloxy-piperidine-1-carboxylic acid benzyl ester are reacted with 300 ml of BBN-H (4N in tetrahydrofuran) at reflux for 48 hours and then with 5.04 ml ethanolamine. The title compound is obtained as an orange oil. Rf = 0.16 (EtOAc - heptane 1:2).

### e) 4-(4-Methoxycarbonyl-phenyl)-3-[4-(3-methoxy-propyl)-3-oxo-3,4-dihydro-2H-benzo[1,4]oxazin-6-ylmethoxy]-5-triisopropylsilanyloxy-piperidine-1-carboxylic acid benzyl ester

According to method D, 7.97 g 3-hydroxy-4-(4-methoxycarbonyl-phenyl)-5-triisopropylsilanyloxy-piperidine-1-carboxylic acid benzyl ester are reacted with 4.486 g 6-chloromethyl-4-(3-methoxy-propyl)-4H-benzo[1,4]oxazin-3-one. The title compound is obtained as an orange oil. Rf = 0.25 (EtOAc- heptane 1:2); Rt = 6.81.

### f) 3-Hydroxy-4-(4-methoxycarbonyl-phenyl)-5-triisopropylsilanoxy-piperidine-1-carboxylic acid benzyl ester

An autoclave under argon containing 80 ml N,N-dimethylformamide and 60 ml methanol is loaded with 0.402 g 1,3-bis(diphenylphosphino)propane and 0.217 g palladium(II)acetate. The mixture is stirred for 20 minutes at room temperature and then 11.95 g 3-hydroxy-4-(4-trifluoromethanesulphonyloxy-phenyl)5-triisopropylsilanyloxy-piperidine-1-carboxylic acid benzyl ester and 5.469 g triethylamine are added. The mixture is stirred for 3 hours under an atmosphere of 5 bars of carbon monoxide at 70°C and then cooled to room temperature. A solution of 0.109 palladium(II)acetate and 0.201 g 1,3-bis(diphenylphosphino)propane in 40 ml N,N-dimethylformamide and 30 ml methanol is added and the mixture is stirred for 3 hours under an atmosphere of 5 bars of carbon monoxide. The reaction mixture is cooled to room temperature, concentrated under reduced pressure by distillation of methanol, poured Into 200 ml water and extracted with 2×250 ml tert-butylmethylether. The combined organic phases are washed with 50 ml brine, dried over sodium sulphate and evaporated. The residue is purified by means of flash column chromatography (SiO2 60F) to provide the title compound as a colorless oil. Rf = 0.31 (EtOAc - heptane 2:3). Rt = 6.36.

### g) 3-Hydroxy-4-(4-trifluoromethanesulphonyloxy-phenyl)-5-triisopropylsilanyloxy-piperidine-1-carboxylic acid benzyl ester

Triethylamine (3.05 ml) and 7.66 g N-phenyl-bis(trifluoromethanesulphonylimide) are added to a solution of 10 g 3-hydroxy-4-(4-hydroxy-phenyl)-5-triisopropylsilanyloxy-piperidin-1-carbonic acid benzyl ester (example 1 e) in 80 ml of dry dichloromethane. The mixture is stirred at room temperature for 4 hours and evaporated under reduced pressure. The residue is purified by means of flash column chromatography (Si02 60F) to provide the title compound as a yellow oil. Rf = 0.17 (EtOAc- heptane 1:3). Rt = 6.60.

### h) 2-(2,5-Difluoro-phenoxy)-ethanol

According to method J, 1.31 g [2-(2,5-difluoro-phenoxy)-ethoxy]-triisopropyl-silane are reacted to afford the title compound as a yellowish oil. Rf = 0.23 (EtOAc - heptane 1:1); Rt = 3.18.

### i) 12-(2,5-Difluoro-phenoxy)-ethoxy]-triisopropyl-silane

A mixture of 0.800 g 2,5-difluorophenol, 1.65 g potassium carbonate and 1.98 g 1-iodo-2-(triisopropylsilyloxy)ethane (CAS 93550-77-7) in 10 ml acetone is stirred at 80°C for 30 hours. The mixture is poured into 75 ml saturated sodium hydrogencarbonate solution and extracted with 2x75 ml tert-butylmethylether. The combined organic phases are washed with 75 ml brine, dried over sodium sulphate and evaporated. The residue is purified by means of flash column chromatography (SiO2 60F) to provide the title compound as a yellowish oil. Rf = 0.75 (EtOAc - heptane 1:10). Rt = 6.67.

According to the process described in Example 22, the following compounds are prepared in an analogous manner:

### Example

26 4-{4-[2(S)-(2,5-Difluoro-phenoxy)-propoxymethyl]-phenyl}-5-[4-(3-methoxypropyl)-3,4-dihydro-2H-benzo[1,4]oxazin-6-ylmethoxy]-piperidin-3-ol

A mixture of 0.069 g 4-{4-[2(S)-(2,5-difluoro-phenoxy)-propoxymethyl]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester, 40% aqueous potassium hydroxide solution (2 ml), methanol (2 ml) and dioxane (3 ml) is heated to 100°C for 14 hours in a sealed tube. The reaction mixture is cooled to room temperature, poured into saturated aqueous sodium bicarbonate solution (20 ml) and washed with tert-butylmethylether (2 x 50 ml). The combined organic phases are washed with water (15 ml), brine (10 ml), dried over sodium sulfate and evaporated under reduced pressure. The residue is purified by means of flash column chromatography (SiO₂ 60F) to provide the title compound.

The starting materials are prepared in the following way:

### a) (S)-2-(2,5-Difluoro-phenoxy)-propan-1-ol

According to method K, 1.33 g (S)-2-(2,5-difluoro-phenoxy)propionic acid ethyl ester are reacted at 60°C for 5 hours to afford the title compound as a colorless oil. Rf = 0.21 (EtOAc - heptane 1:3); Rt = 3.51.

### b) (S)-2-(2,5-Difluoro-phenoxy)-propionic acid ethyl ester

According to method 1, 2.00 g ethyl L-(-)-methanesulphonyl lactate and 1.256 g 2,5-difluorophenol are reacted at room temperature for 48 hours to afford the title compound as a colorless oil. Rf = 0.39 (EtOAc - heptane 1:5); Rt = 4.53.
35 4-{4-[2-(2,5-Dichloro-phenoxy)i-ethoxymethyl]-phenyl}-5-[4-(3-methoxy-propyl)-3,4-dihydro-2H-benzo[1,4]oxazin-6-ylmethoxy]-piperidin-3-ol

### Example 27

### 4-{4-[2-(2-Chloro-benzyloxy)-ethoxy]-phenyl}-5-[4-(3-methoxy-propyl)-3,4-dihydro-2H-benzo[1,4]oxazin-6-ylmethoxy]-piperidin-3ol

A mixture of 0.125 g 4-{4-[2-(2-chloro-benzyloxy)-ethoxy]phenyl}-3-hydroxy-5-[4-(3-methoxypropyl),3,4-dihydro-2H-benzo[1,4]oxazin-6-ylmethoxy]-piperidine-1-carboxylic acid benzyl ester, 40% aqueous potassium hydroxide solution (8 ml) and methanol (8 ml) is heated to 105°C for 6 hours in a sealed tube. The reaction mixture is cooled to room temperature and diluted with water (40 ml) and ethyl acetate (40 ml). The layers are separated and the aqueous layer is extracted once with ethyl acetate. The combined organics are dried over sodium sulphate and concentrated. The residue is purified by means of flash column chromatography (SiO₂ 60F) to provide the title compound.

The starting materials are prepared analogous to example 1 starting from 2-(2-chlorobenzyloxy)-ethanol (CAS 1199-30-0).

### Example 28

### Dimethyl-carbamic acid 4-(4-methoxy-phenyl)-5-[4-(3-methoxy-propyl)-3,4-dihydro-2H-benzo[1,4]oxazin-6-ylmethoxy]-piperidin-3-yl ester

The title compound was prepared according to method C starting from 0.029 g of 3-dimethylcarbamoyloxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:

### a) 3-Dimethylcarbamoyloxy-4-(4-methoxy-phenyl)-5[4-(3-methoxy-propyl)-3,4-dihydro-2H-benzo[1,4]oxazin-6-ylmethoxy]-piperidine-1-carboxylic acid benzyl ester

Sodium hydride (0.007 g) is added to a solution of 0.105 g 3-hydroxy-4-(4-methoxy-phenyl)-5-[4-(3-methoxy-propyl)3,4-dihydro-2H-benzo[1,4]oxazin-6-ylmethoxy]-piperidine-1-carboxylic acid benzyl ester (example 3a) in 2 ml of tetrahydrofuran. The mixture is stirred for 30 minutes at room temperature. N,N-Dimethylcarbamoyl chloride (0.029 ml) is added and the mixture is heated to reflux for 2 hours. Additional sodium hydride (0.007 g) and N,N-dimethylcarbamoyl chloride (0.010 ml) are added and the mixture heated to reflux for one more hour. The reaction mixture is quenched with water and is extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless oil. Rf = 0.38 (EtOAc - heptane 3: 1); Rt = 5.26.

### Example 29

### 4-{4-[1-(3-Fluoro-phenyl)-pyrrolidin-3(R)-yloxy]-phenyl}-5-[4-(3-methoxy-propyl)-3,4-dihydro-2H-benzo[1,4]-oxazin-6-ylmethoxy]-piperidin-3-ol

The title compound was prepared according to method C starting from 0.022 g of 4-{4-[1-(3-fluor-phenyl)-pyrrolidin-3-yloxy]-phenyl}-3(R)-hydroxy-5-[4-(3-methoxy-propyl)-3,4-dihydro-2H-benzo[1,4]-oxazin-6-ylmethoxy]-piperidine-1-carboxylic acid benzyl ester.

The starting material is prepared as follows:

### a) 4-{4-[1-(3-Fluor-phenyl)-pyrrolidin-3-yloxy]-phenyl}-3(R)-hydroxy-5-[4-(3-methoxypropyl)-3,4-dihydro-2H-benzo[1,4]-oxazin-6-ylmethoxy]-piperidine-1-carboxylic acid benzyl ester

A solution of 0.025 ml diisopropylazodicarboxylate in 1 ml of tetrahydrofuran is added dropwise to a solution of 0.045 g 4-{4-[1-(3-fluor-phenyl)-pyrrolidin-3-yloxy]-phenyl}-3-hydroxy-5-[4-(3-methoxy-propyl)-3,4-dihydro-2H-benzo[1,4]-oxazin-6-ylmethoxy]-piperidine-1-carboxylic acid benzyl ester (example 1a), 0.015 g benzoic acid and 0.032 g triphenylphosphine in 1 ml of tetrahydrofuran The reaction solution is stirred for 3 hours at room temperature and is then concentrated under reduced pressure. The residue is purified through a short pad of SiO₂. The crude material is then dissolved in 2.5 ml of methanol/tetrahydrofuran 4:1. Potassium carbonate (0.034 g) is added to the solution and the mixture is stirred for 16 hours at room temperature. The reaction mixture is quenched with water and extracted with tert-butyl methyl ether (2x). The combined organics are washed with brine, dried (sodium sulphate) and concentrated under reduced pressure. The title compound is obtained as a colorless glass and can be used without further purification. Rf = 0.51 (EtOAc - heptane 2:1); Rt = 5.59.

### Example 30

### Acetic acid 4-(4-methoxy-phenyl)-5-[4-(3-methoxy-propyl)-3,4-dihydro-2H-benzo[1,4]oxazin-6-ylmethoxy]-piperidin-3-yl ester

The title compound is prepared according to method C starting from 0.038 g of 3-acetoxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:

### a) 3-Acetoxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester

Acetic anhydride (0.018 ml) is added to a solution of 0.051 g 3-hydroxy-4-(4-methoxyphenyl)-5-[4-(3-methoxy-propyl)-3,4-dihydro-2H-benzo[1,4]oxazin-6-ylmethoxy]-piperidine-1-carboxylic acid benzyl ester (example 3a), 0.002 g N,N-dimethylaminopyridine and 0.036 ml triethylamine in 2 ml of dichloromethane The mixture is stirred for 24 hours at room temperature. The reaction mixture is quenched with water and extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless oil. Rf = 0.22 (EtOAc - heptane 1:1); Rt = 5.31.

### Example 31

### 4-{4-[1-(3-Fluoro-phenyl)pyrrolidine-yloxy]-phenyl}-5-[4-(3-methoxy-propyl)-3,4-dihydro-2H-benzo[1,4]oxazin-6-ylmethoxy]-piperidin-3(R)-ylamine

The title compound is prepared according to method C starting from 0.106 g of 3(R)-azido-4-{4-[1-(3-fluoro-phenyl)-pyrrolidin-3-yloxy]-phenyl}-5-[4-(3-methoxy-propyl)-3,4-dihydro-2H-benzo[1,4]-oxazin-6-ylmethoxy]-piperidine-1-carboxylic acid benzyl ester.

The starting materials are prepared as follows:

### a) 3(R)-Azido-4-{4-[1-(3-fluoro-phenyl)-pyrrolidin-3-yloxy]-phenyl}-5-[4-(3-methoxy-propyl)-3,4-dihydro-2H-benzo[1,4]-oxazin-6-ylmethoxy]-piperidine-1-carboxylic acid benzyl ester

A mixture of 0.174 g 4-{4-[1-(3-fluoro-phenyl)pyrrolidin-3-yloxy]-phenyl}-3-methanesulphonyloxy-5-[4-(3-methoxy-propyl)-3,4-dihydro-2H-benzo[1,4]-oxazin-6-ylmethoxy]-piperidine-1-carboxylic acid benzyl ester and 0.141 g sodium azide in 4 ml of dimethylformamide is heated to 100°C for 24 hours. The reaction mixture is poured on water and extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless wax. Rf = 0.39 (EtOAc- heptane 1:1); Rt = 5.98.

### b) 4-{4-[1-(3-Fluoro-phenyl)-pyrrolidin-3-yloxy]-phenyl}-3-methanesulphonyloxy-5-[4-(3-methoxy-propyl-3,4-dihydro-2H-benzo[1,4]-oxazin-6-ylmethoxy]-piperidine-1-carboxylic acid benzyl ester

Methanesulphonyl chloride (0.040 ml) is added to a solution of 0.261 g 4-{4-[1-(3-Fluorphenyl)-pyrrolidin-3-yloxy]-phenyl}-3-hydroxy-5-[4-(3-methoxy-propyl)-3,4-dihydro-2H-benzo[1,4]-oxazin-6-ylmethoxy]-piperidine-1-carboxylic acid benzyl ester (example 1a), 0.090 ml triethylamine and 0.005 g N,N-dimethylaminopyridine in 1 ml of dichloromethane. The reaction mixture is stirred for 1 hour at room temperature and is then poured on saturated aqueous sodium bicarbonate solution and is extracted with tert-butyl methyl ether (2x). The combined organics are washed with brine, dried (sodium sulphate) and concentrated under reduced pressure. The title compound is obtained as beige foam and can be used for the next step without purification. Rt = 5.76.

### Example 32

### 2,2-Dimethyl-propionic acid 4-(4-methoxy-phenyl)-5-[4-(3-methoxy-propyl)-3,4-dihydro-2H-benzo[1,4]oxazin-6-ylmethoxy]-piperidin-3-yl ester

The title compound is prepared according to method C starting from 0.042 g of 3-(2,2-dimethyl-propionyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:

### a) 3-(2,2-Dimethyl-propionyloxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester

Pivaloyl chloride (0.020 ml) is added to a solution of 0.052 g 3-hydroxy-4-(4-methoxy-phenyl)-5-[4-(3-methoxy-propyl)-3,4dihydro-2H-benzo[1,4]oxazin-6-ylmethoxy]-piperidine-1-carboxylic acid benzyl ester (example 3a) and 0.003 g N,N-dimethylaminopyridine in 2 ml of pyridine. The mixture is stirred for 4 hours at reflux and then quenched with saturated aqueous sodium bicarbonate solution and is extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless oil. Rf = 0.31 (EtOAc - Heptan 1:1); Rt = 5.89.

According to the process described in Example 32, the following compound is prepared in an analogous manner:

### Example

33 Isobutyric acid 4-(4-methoxy-phenyl)-5-[4-(3-methoxy-propyl)-3,4-dihydro-2H-benzo[1,4)oxazin-6-ylmethoxy]-piperidin-3-yl ester

### Example 34

### 4{4-[3-(2,5-Difluoro-phenyl)-propoxymethyl]-phenyl}-5-[4-(3-methoxy-propyl)-3,4-dihydro-2H-benzo[1,4]oxazin-6-ylmethoxy]-piperidin-3-ol

The title compound is prepared according to example 26 starting from 0.160 g of 4-{4-[3-(2,5-difluoro-phenyl)-propoxymethyl]-phenyl}-3-hydroxy-5-[4-(3-methoxy-propyl)-3,4-dihydro-2H-benzo[1,4]oxazin-6-ylmethoxy]-piperidine-1-carboxylic acid benzyl ester.

The starting materials are prepared analogous to example 22 starting from 3-(2,5-difluorophenyl)-propan-1-ol.

The starting material is prepared as follows:

### a) 3-(2,5-Difluoro-phenyl)-propan-1-ol

According to method K, 2.10 g 3-(2,5-difluorophenyl)propionic acid (CAS: 130408-15-0) are reacted to afford the title compound as a colorless oil. Rf = 021 (EtOAc - heptane 1:3); Rt = 3.54.

### Example 36

### 6-[4-(4-Methoxy-phenyl)-5-phenoxy-piperidin-3-xloxymethyl]-4-(3 methoxy-propyl)-3,4-dihydro-2H-benzo[1,4]oxazine

The title compound is prepared according to method C starting from 0.023 g of 3-(4-chlorophenoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester.

The starting material is prepared as follows:

### a) 3-(4-Chloro-phenoxy)-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester

Sodium hydride (0.012 g) is added to a cooled (0°C) solution of 0.050 g 3-hydroxy-4-(4-methoxy-phenyl)-5-[4-(3-methoxy-propyl)-3,4-dihydro-2H-benzo[1,4]oxazin-6-ylmethoxy]-piperidine-1-carboxylic acid benzyl ester (example 3a) in 2 ml of N,N-dimethylformamide. The mixture is stirred for 15 minutes at 0°C and 1-chloro-4-fluorobenzene (0.013 ml) is added. The mixture is heated to 70°C for 2 h. The reaction is quenched with saturated aqueous sodium bicarbonate solution and is extracted with tert-butyl methyl ether (2x). The combined organics are washed with brine, dried (sodium sulphate) and concentrated under reduced pressure. The residue is dissolved in ethyl acetate (10 ml). Saturated aqueous sodium carbonate solution(10 ml) is added and the mixture cooled to 0°C. Benzyl chloroformate (0.015 ml) is added and the reaction mixture is then stirred vigorously for 30 minutes. The layers are separated and the aqueous layer is extracted with ethyl acetate (1x). The combined organics are washed with brine, dried over sodium sulphate and concentrated. The residue is purified by means of flash column chromatography (SiO₂ 60F) to provide the title compound as a colorless wax. Rf = 0.12 (EtOAc - heptane 1:2); Rt = 5.94.

### Example 37

### 4-{4-[1-(3-Fluoro-phenyl)pyrrolidine-yloxy]-phenyl}-5-[4-(3-methoxy-propyl)-3,4-dihydro-2H-benzo[1,4]oxazin-6-ylmethoxy]-piperidin-3(S)-ylamine

The tittle compound is prepared analogous to example 31 starting from 4-{4-[1-(3-fluorphenyl)-pyrrolidin-3-yloxy]-phenyl}-3(R)-hydroxy-5-[4-(3-methoxy-propyl)-3,4-dihydro-2H-benzo[1,4]-oxazin-6-ylmethoxy]-piperidine-1-carboxylic acid benzyl ester (example 29a).

### Example 38

### {4-{4-[1-(3-Fluorophenyl)-pyrrolidin-yloxy]-phenyl}-5-[4-(3-methoxy-propyl)-3,4-dihydro-2H-benzo[1,4]oxazin-6-ylmethoxy]-piperidin-3-yl methyl-amine

The title compound is prepared according to method C starting from 0.046 g of 3-(benzyloxycarbonyl-methyl-amino)-4-{4-[1-(3-fluoro-phenyl)-pyrrolidin-3-yloxy]-phenyl}-5-[4-(3-methoxy-propyl)-3,4-dihydro-2H-benzo[1,4]-oxazin-6-ylmethoxy]-piperidine-1-carboxylic acid benzyl ester.

The starting materials are prepared as follows:

### a) 3-(Benzyloxycarbonyl-methyl-amino)-4-{4-[1-(3-fluoro-phenyl)-pyrrolidin-3-yloxy]-phenyl} -5-[4-(3-methoxy-propyl)-3,4-dihydro-2H-benzo[1,4]-oxazin-6-ylmethoxy]-piperidine-1-carboxylic acid benzyl ester

Sodium hydride (0.011 g) is added to a cooled (0°C) solution of 0.087 g 3-benzyloxycarbonylamino-4-{4-[1-(3-fluoro-phenyl)-pyrrolidin-3-yloxy]phenyl}-5-[4-(3-methoxy-propyl)-3,4-dihydro-2H-benzo[1,4]-oxazin-6-ylmethoxy]-piperidine-1-carboxylic acid benzyl ester in 2 ml of N,N-dimethylformamide. The mixture is stirred for 15 minutes at 0°C and then methyl iodide (0.023 ml) is added. The mixture is stirred at room temperature for 16 hours. The reaction is quenched with saturated aqueous sodium bicarbonate solution and is extracted with tert-butyl methyl ether (2x). The combined organics are washed with brine, dried (sodium sulphate) and concentrated under reduced pressure. The residue is purified by means of flash column chromatography (SiO₂ 60F) to provide the title compound as a colorless wax. Rf = 0.15 (EtOAc- heptane 1:1); Rt = 6.09.

### b) 3-Benzyloxycarbonylamino-4-{4-[1-(3-fluoro-phenyl)-pyrrolidin-3-yloxy]-phenyl} -5-[4-(3-methoxy-propyl)-3,4-dihydri-2H-benzo[1,4]-oxazin-6-ylmethoxy]-piperidine-1-carboxylic acid benzyl ester

Saturated aqueous sodium carbonate solution (3 ml) is added to a solution of 0.090 g 4-{4-[1-(3-fluoro-phenyl)pyrrolidin-yloxy]-phenyl}-5-[4-(3-methoxy-propyl)-3,4-dihydro-2H-benzo[1,4]oxazin-6-ylmethoxy]-piperidin-3(S)-ylamine (example 37) in ethyl acetate (3 ml) and the mixture cooled to 0°C. Benzyl chloroformate (0.056 ml) is added and the reaction mixture is then stirred vigorously for 30 min. The layers are separated and the aqueous layer is extracted with ethyl acetate (1x). The combined organics are washed with brine, dried over sodium sulphate and concentrated. The residue is purified by means of flash column chromatography (SiO₂ 60F) to provide the title compound as a colorless wax. Rf = 0.23 (EtOAc - heptane 1:1); Rt = 5.88.

### Example 39

### (2-{4-{4-[1-(3-Fluoro-phenyl)-pyrrolidin-3-yloxy]-phenyl}-5-[4-(3-methoxy-propyl)-3,4-dihydro-2H-benzo[1,4]oxazin-6-ylmethoxy]-piperidin-3-yloxy}-ethyl)-methyl-amine

A suspension of 0.500 g N-{2-[4-{4-[1-(3-fuoro-phenyl)-pyrrolidin-3-yloxy]-phenyl}-5-[4-(3-methoxy-propyl)-3,4dihydro-2H-benzo[1,4]-oxazin-6-ylmethoxy]-1-(toluene-4-sulfonyl)-piperidin-3yloxy]-ethy}-4,N-dimethyl-benzenesulfonamide and 0.341 g sodium dihydrogenphosphate monohydrate in 14 ml of methanol/tetrahydrofuran 6:1 is treated portion wise with 3.200 g of Na/Hg over a period of 2 hours. After the last portion is added the reaction mixture is stirred for 16 hours at room temperature. The mixture is filtered over Hyflo and the filtrate is concentrated under reduced pressure. The residue is taken up in a 2:1 mixture of dichloromethane/saturated aqueous sodium bicarbonate solution. The layers are separated and the organic layer is washed with water, dried over sodium sulphate and evaporated. The residue is purified by means of flash column chromatography (SiO₂ 60F).

The starting material are prepared as follows:

### a) N-{2-[4-{4-[1-(3-Fluoro-phenyl)-pyrrolidin-3-yloxy]-phenyl}-5-[4-(3-methoxy-propyl)-3,4-dihydro-2H-benzo[1,4]-oxazin-6-ylmethoxy]-1-(toluene-4-sulfonyl)-piperidin-3yloxy]-ethyl}-4,N-dimethyl-benzenesulfonamide

Sodium hydride (0.306 g) is added to a solution of 1.430 g 4-{4-[1-(3-fluoro-phenyl)-pyrrolidin-3-yloxy]-phenyl}-5-[4-(3-methoxy-propyl)-3,4-dihydro-2H-benzo[1,4]oxazin-6-ylmethoxy]-1-(toluene-4-sulfony)-piperidin-3-ol in 10 ml of dry tetrahydrofuran. The mixture is warmed to 50°C and a solution of 4.770 g toluene-4-sulfonic acid 2-[methyl-(toluene-4-sulfonyl)-amino]-ethyl ester (CAS 3559-06-6) In 10 ml of tetrahydrofuran is added. The reaction solution is stirred at 50°C for 20 hours. The solution is cooled to room temperature, diluted with tert-butyl methyl ether and washed with saturated aqueous sodium bicarbonate solution and brine, dried (sodium sulphate) and concentrated under reduced pressure. The residue is purified by means of flash column chromatography (SiO₂ 60F) to provide the title compound as a colorless foam. Rf = 0.47 (EtOAc- Heptan 2:1); Rt = 5.95.

### b) 4-{4-[1-fluoro-phenyl)-pyrrolidin-3-yloxy]-phenyl}-5-[4-(3-methoxy-propyl)-3,4-dihydro2H-benzo[1,4]oxazin-6-ylmethoxy]-1-(toluene-4-sulfonyl)-piperidin-3-ol

Saturated aqueous sodium carbonate solution (25 ml) is added to a solution of 1.280 g 4-{4-(1-(3-fluoro-phenyl)-pyrrolidin-3-yloxy]-phenyl}-[4-(3-methoxy-propyl)-3,4-dihydro-2H-benzo[1,4]-oxazin-6-ylmethoxy]-piperidin-3-ol_(example 1)_in ethyl acetate_(25 ml) and the mixture cooled to 0°C. 4-Toluenesulfonyl chloride (0.460 g) is added and the reaction mixture is then stirred vigorously for 1 hour. The layers are separated and the aqueous layer is extracted with ethyl acetate (1x). The combined organics are washed with brine, dried over sodium sulphate and concentrated. The residue is purified by means of flash column chromatography (SiO₂ 60F) to provide the title compound as a colorless foam. Rf = 0.16 (EtOAc - heptane 1:1); Rt = 5.40.

### Example 40

### 4-(4-Isobutyl-phenyl)-5-[4-(3-methoxy-propyl)-3,4-dihydro-2H-benzo[1,4]oxazin-6-ylmethoxy]-piperidin-3-ol

To a stirred solution of 102.5 mg 3-hydroxy-4-{4-[1-(2-methoxy-acetoxy)-2-methyl-propyl]-phenyl}-5-[4-(3-methoxy-propyl)-3,4-dihydro-2H-benzo[1,4]oxazin-6-ylmethoxy]-piperidine-1-carboxylic acid benzyl ester (diastereoisomeric mixture) in 4 ml ethanol are added 10µl ethanolamine and Pd/C (10%, Engelhard). The mixture is hydrogenated for 14 hours at 0°C. Then the reaction mixture is filtered through a pad of celite and evaporated under reduced pressure. The residue is purified by means of flash column chromatography (SiO₂ 60F) to , provide the title compound as a colorless oil. Rf = 0.22 (dichloromethane-methanol-ammonia 200:20:1); Rt = 4.28.

The starting materials are prepared as follows:

### a) 3-Hydroxy-4-{4-[1-(2-methoxy-acetoxy)-2-methyl-propyl]-phenyl}-5[4-(3-methoxypropyl)-3,4-dihydro-2H-benzo[1,4]oxazin-6-ylmethoxy]piperidine-1-carboxylic acid benzyl ester (diastereoisomeric mixture)

To a stirred solution of 182.3 mg 4-{4-[1-(2-methoxy-acetoxy)-2-methyl-propyl]-phenyl}-3-[4-(3-methoxy-propyl)-3,4-dihydro-2H-benzo[1,4]oxazin-6-ylmethoxy]-5-triisopropylsilanyloxypiperidine-1-carboxylic acid benzyl ester (diastereoisomeric mixture) in 1.5 ml tetrahydrofuran is added 0.58 ml tetrabutylammoniumfluoride (1M in tetrahydrofuran). The reaction mixture is stirred for 2 hours at room temperature. The reaction mixture is quenched with water and extracted with ethyl acetate (2x). The combined organics were washed with brine, dried (sodium sulphate) and concentrated under reduced pressure. The residue is purified by means of flash column chromatography (SiO₂ 60F) to provide the title compound as a white foam. Rf = 0.18 (EtOAc - heptane 2:1); Rt = 5.11.

### b) 4-{4-[1-(2-Methoxy-acetoxy)-2-methyl-propyl]-phenyl}-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester (diastereoisomeric mixture)

To a stirred solution of 194 mg 4-[4-(1-Hydroxy-2-methyl-propyl)-phenyl]-3-[4-(3-methoxypropyl)-3,4-dihydro-2H-benzo[1,4]oxazin-6-ylmethoxy]-5-triisopropylsilanyloxy-piperidine-1-carboxylic acid benzyl ester (diastereoisomeric mixture) in 3.5 ml toluene are added at 0°C 53 µl pyridine, 3.1 mg 4-dimethylaminopyridine and 56 µl methoxyacetyl chloride. The solution is allowed to warm to room temperature and is stirred for 2h. The reaction mixture is quenched with water and extracted with ethyl acetate (2x). The combined organics are washed with brine, dried (sodium sulphate) and concentrated under reduced pressure. The residue is purified by means of flash column chromatography (SiO₂ 60F) to provide the title compound as a light yellow oil. Rf = 0.26 (EtOAc- heptane 2:3).

### c) 4-[4-(1-Hydroxy-2-methyl-propyl)-phenyl]-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester (diastereoisomeric mixture)

To a stirred solution of 320 mg 4-(4-Formyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester in 5 ml tetrahydrofuran are added at 0°C 0.49 ml isopropylmagnesium bromide (1M in tetrahydrofuran). After stirring for 1 hour, the reaction mixture is allowed to warm to room temperature. The reaction mixture is quenched with 1M HCl and extracted with ethyl acetate (2x). The combined organics are washed with brine, dried (sodium sulphate) and concentrated under reduced pressure. The residue is purified by means of flash column chromatography (SiO₂ 60F) to provide the title compound as a yellow oil. Rf = 0.27 (EtOAc - heptane 1:2).

### d) 4-(4-Formyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester

To a stirred solution of 230 mg o-iodoxy benzoic acid (IBX) in 3 ml dimethylsulfoxide is added a solution of 500 mg 4-(4-hydroxymethyl-phenyl)-3-[4-(3-methoxy-propyl)-3,4-dihydro-2H-benzo[1,4]oxazin-6-ylmethoxy]-5-triisopropylsilanyloxy-piperidine-1-carboxylic acid benzyl ester (example 22) in 3 ml dimethylsulfoxide at room temperature. The reaction mixture is stirred for 30 minutes and then 0.5N NaOH is added. The resulting mixture is extracted with with tert-butyl methyl ether (2x) and the combined organics are washed with brine, dried (sodium sulphate) and concentrated under reduced pressure. The residue is purified by means of flash column chromatography (SiO₂ 60F) to provide the title compound as a yellow oil. Rf = 0.54 (EtOAc - heptane 1:1); Rt = 6.09.

### Example 41

### 6-{4-{4-[1-(3-Fluoro-phenyl)-pyrrolidin-3-yloxy]-phenyl}-5-[2-(1H-tetrazol-5-yl)-ethoxy]-piperidin-3-yloxymethyl}-4-(3-methoxy-propyl)-3,4-dihydro-2H-benzo[1,4]oxazine

The title compound is prepared according to example 39 starting from 0.600 g 6-[4-{4-[1-(3-fluoro-phenyl)-pyrrolidin-3-yloxy]-phenyl}-5-[2-(1H-tetrazol-5-yl)-ethoxy]-1-(toluene-4-sulfonyl)-piperidin-3-yloxymethy]-4-(3-methoxy-propyl)-3,4-dihydro-2H-benzo[1,4]oxazine.

The starting materials are prepared as follows:

### a) 6-[4-{4-[1-(3-Fluoro-phenyl)-pyrrolidin-3-yloxy]-phenyl}-5-[2-(1H-tetrazol-5-yl)-ethoxy]-1-(toluene-4-sulfonyl)-piperidin-3-yloxymethyl]-4-(3-methoxy-propyl)-3,4-dihydro-2H-benzo[1,4]oxazine

Dibutyltin oxide (0.0686 g) is added to a solution of 1.20 g 3-[4-{4-[1-(3-Fluoro-pheny)-pyrrolidin-3-yloxy]-phenyl}-5-[4-(3-methoxy-propyl)-3,4-dihydro-2H-benzo[1,4]oxazin-6-ylmethoxy]-1-(toluene-4-sulfonyl)-piperidin-3-yloxy]-propionitrile and 2.36 g trimethylsilylazide In 15 ml dry toluene. The mixture is stirred at 100°C for 19 hours and then cooled to room temperature, diluted with 100 ml ethyl acetate and washed with 30 ml 1N HCl. The aqueous phase is extracted with 100 ml ethyl acetate (2x). The combined organic phases are washed with 50 ml water and 50 ml brine, dried over sodium sulphate and evaporated under reduced pressure. The residue is purified by means of flash column chromatography (SiO₂ 60F) to provide the title compound as a colorless oil. Rf = 0.10 (EtOAc); Rt = 5.19.

### b) 3-[4-{4-[1-(3-Fluoro-phenyl)-pyrrolidin-3-yloxy]-phenyl}-5-[4-(3-methoxy-propyl)-3,4-dihydro-2H-benzo[1,4]oxazin-6-ylmethoxy]-1-(toluene-4-sulfonyl)-piperidin-3-yloxy]-propionitrile

0.378 g 2,3,4,6,7,8,9,10-Octahydro-pyrimido[1,2-a]azepine (DBU) is added to a solution of 1.85 g 4-{4-[1-(3-fluoro-phenyl)-pyrrolidin-3-yloxy]-phenyl}5-[4-(3-methoxy-propyl)-3,4-dihydro-2H-benzo[1,4]oxazin-6-ylmethoxy]-1-(toluene-4-sulfonyl)-piperidin-3-ol (example 39b) and 0.658 g acrylonitrile in 12 ml dry acetonitrile in a sealed tube protected from light. The mixture is stirred for 48 hours at 50°C and then cooled to room temperature. 0.658 g acrylonitrile and 0.378 g DBU are added and the reaction mixture is stirred for 24 hours at 50°C. This process is repeated once, then the mixture is evaporated under reduced pressure. The residue is purified by means of flash column chromatography (SiO₂ 60F) to provide the title compound as a colorless oil. Rf = 0.33 (EtOAc - heptane 1:1); Rt = 5.74.

## Claims

1. A compound of the formula (1) 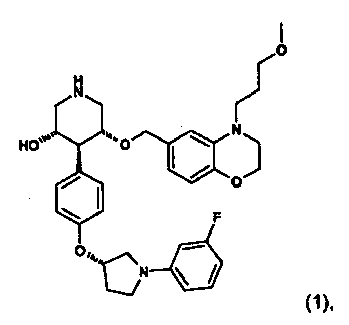 a compound of the formula (2) 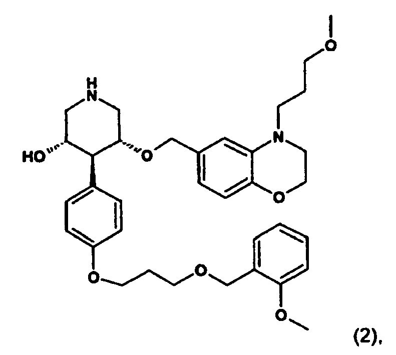 a compound of the formula (3) 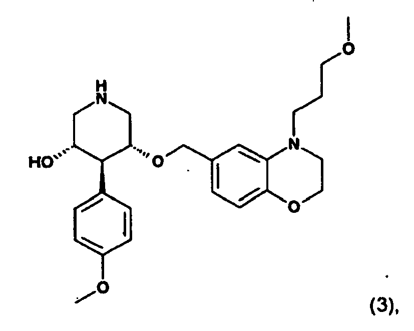 a compound of the formula (4) 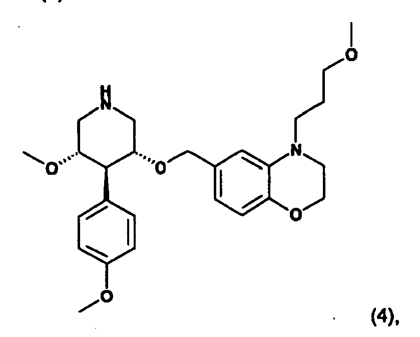 a compound of the formula (5) 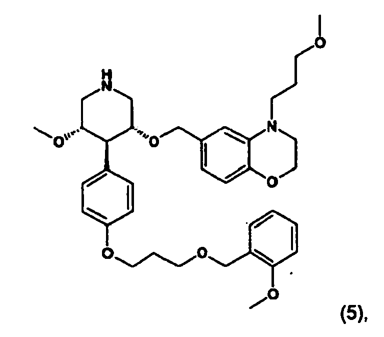 a compound of the formule (6) 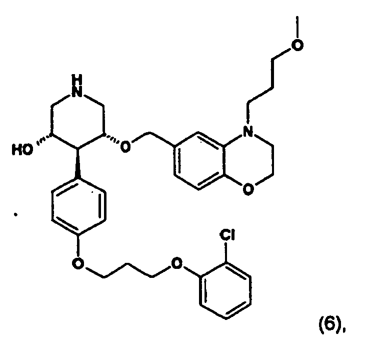 a compound of the formula (7) 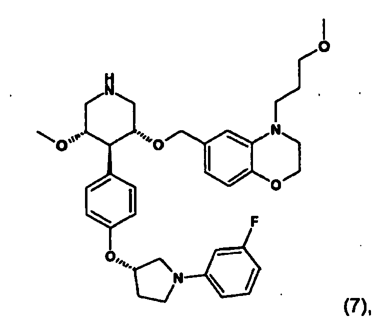 a compound of the formula (8) 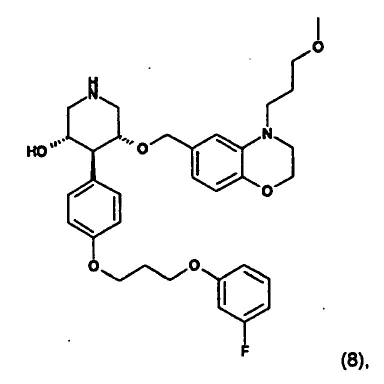 a compound of the formula (9) 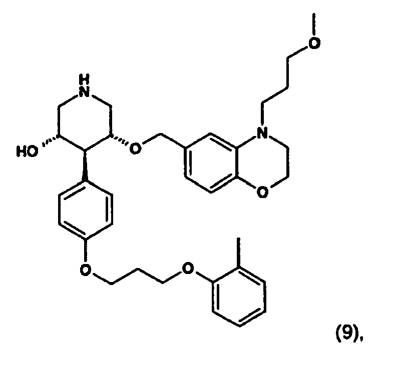 a compound of the formula (10) 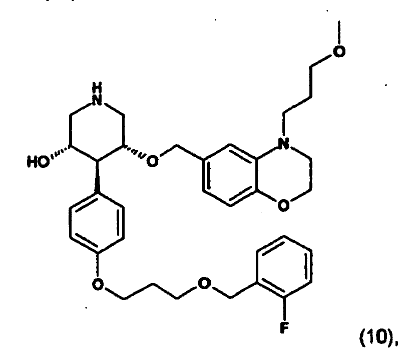 a compound of the formula (11) 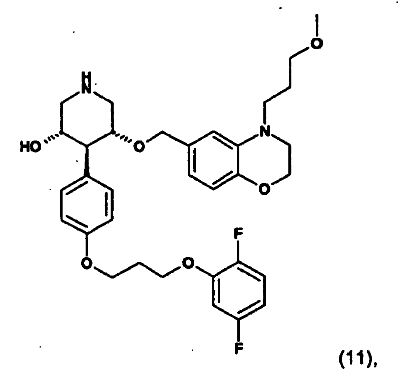 a compound of the formula (12) 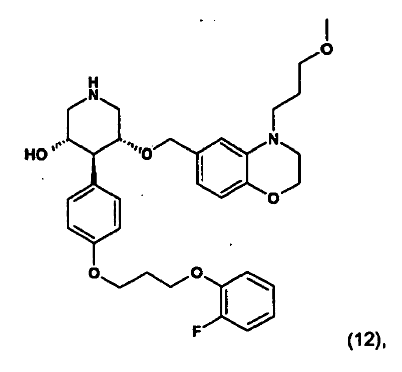 a compound of the formula (13) 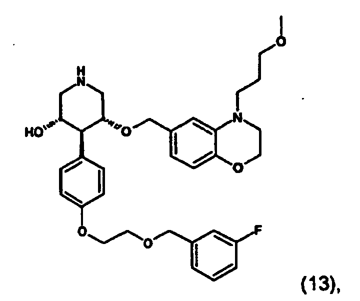 a compound of the formula (14) 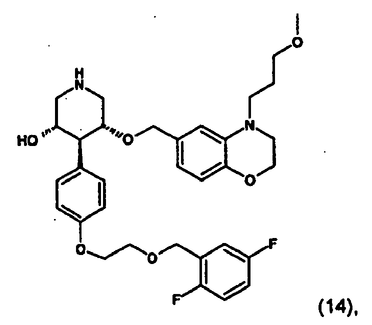 a compound of the formula (15) 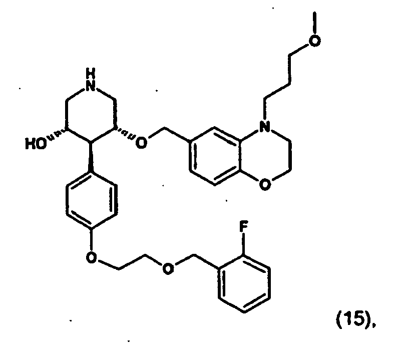 a compound of the formula (16) 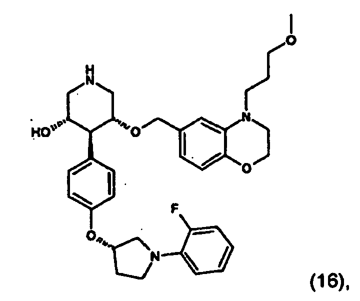 a compound of the formula (17) 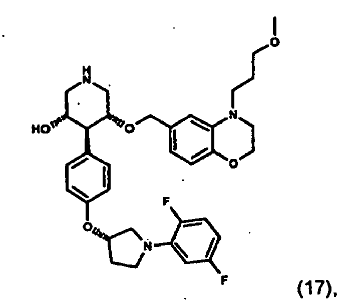 a compound of the formula (18) 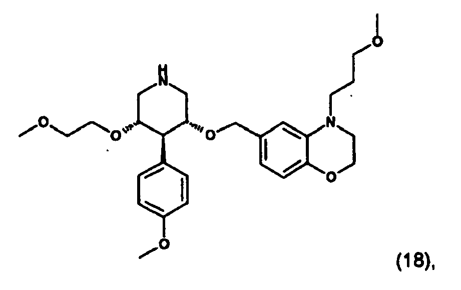 a compound of the formula (19) 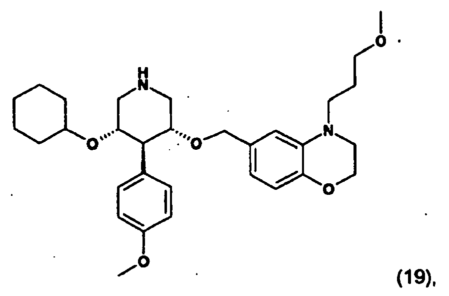 a compound of the formula (20) 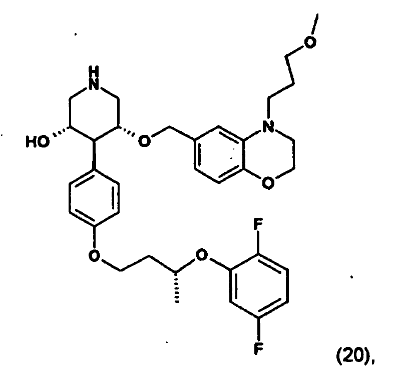 a compound of the formula (21) 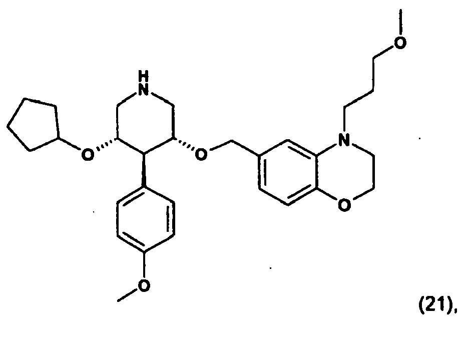 a compound of the formula (22) 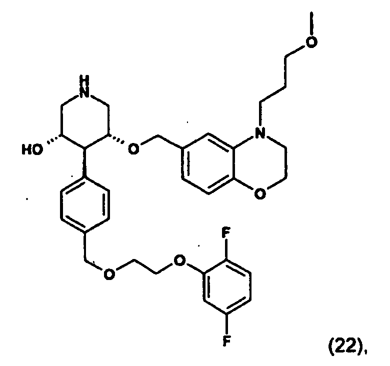 a compound of the formula (23) 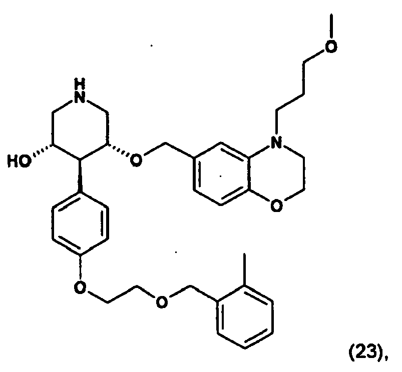 a compound of the formula (24) 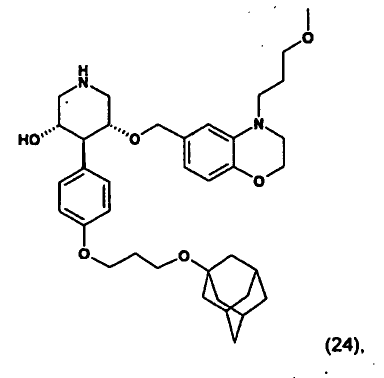 a compound of the formula (25) 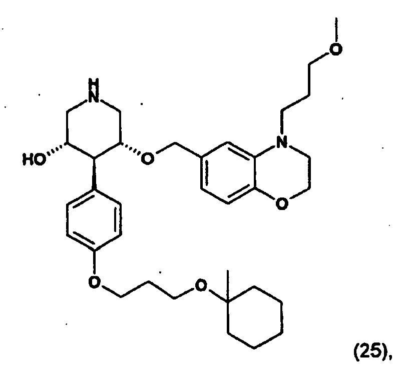 a compound of the formula (26) 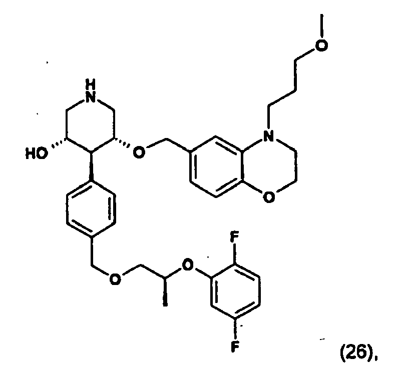 a compound of the formula (27) 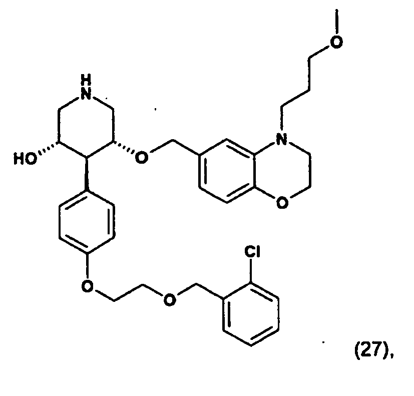 a compound of the formula (28) 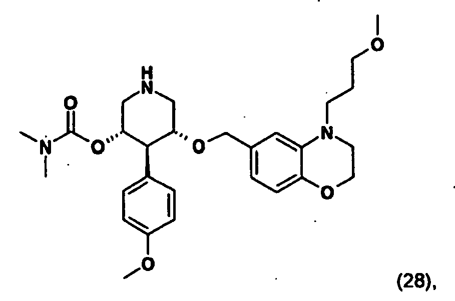 a compound of the formula (29) 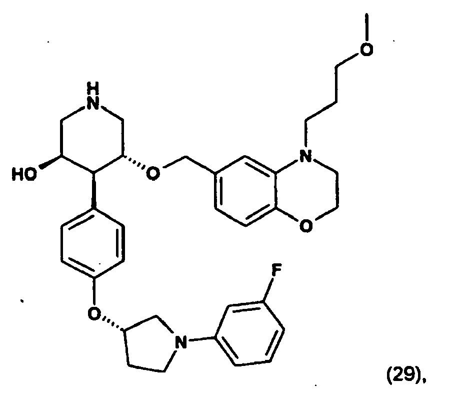 a compound of the formula (30) 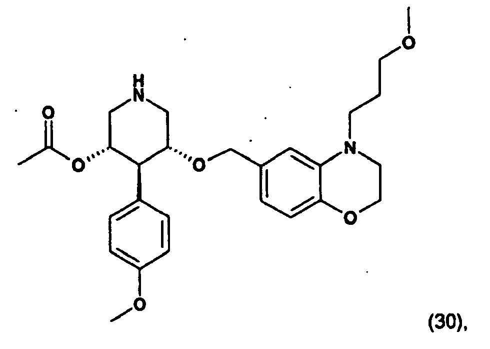 a compound of the formula (31) 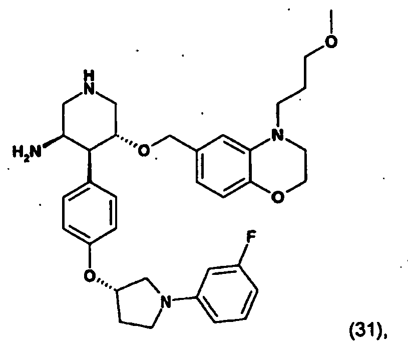 a compound of the formula (32) 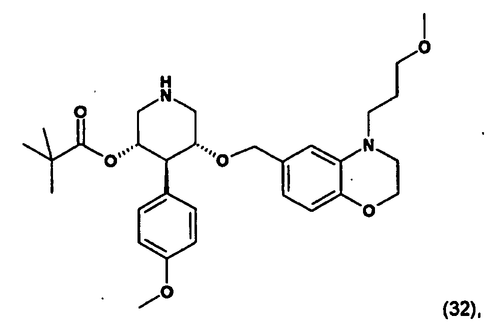 a compound of the formula (33) 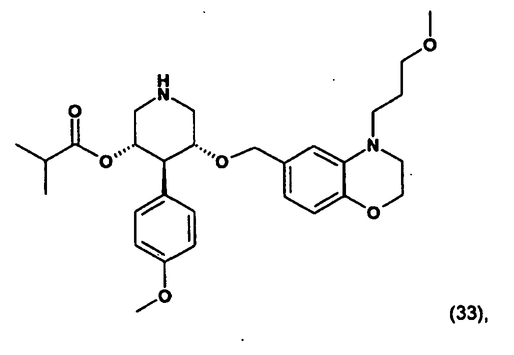 a compound of the formula (34) 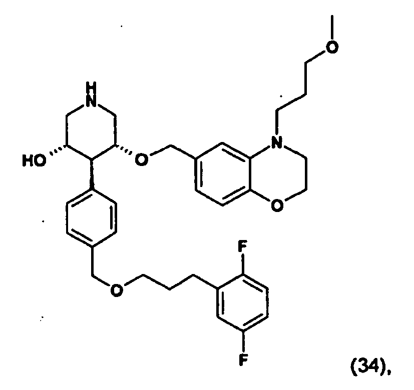 a compound of the formula (35) 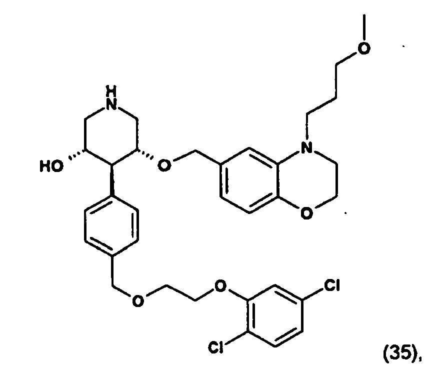 a compound of the formula (36) 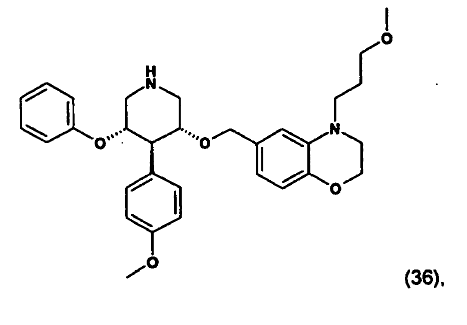 a compound of the formula (37) 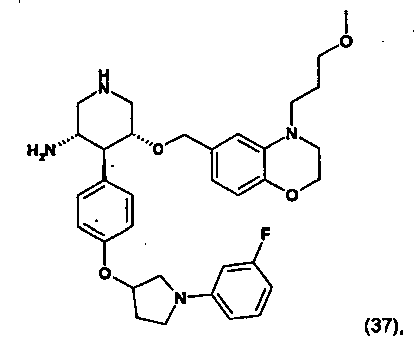 a compound of the formula (38) 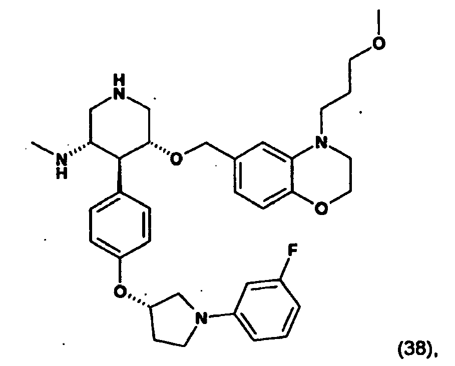 a compound of the formula (39) 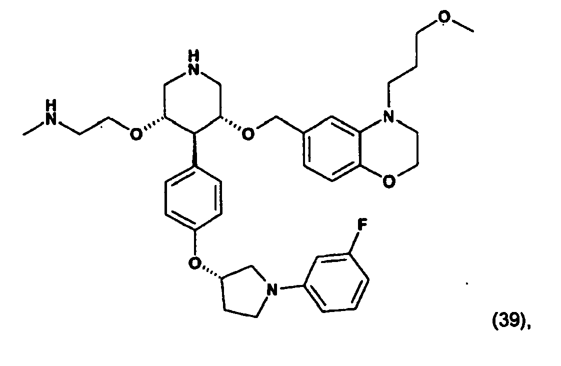 a compound of the formula (40) 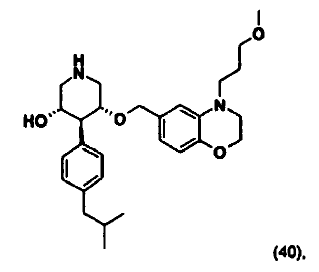 a compound of the formula (41) 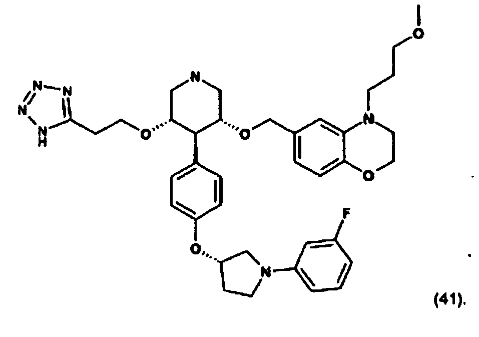 or a pharmaceutically acceptable salt thereof.

2. Any one of the compounds of the formula (1) to formula (41) according to claim 1, or a pharmaceutically acceptable salt thereof, for producing a medicine.

3. Any one of the compounds of the formula (1) to formula (41) according to claim 1, or a pharmaceutically acceptable salt thereof, for use in producing a human medicine for the prevention, for the retardation of progression or for the treatment of hypertension and heart failure, and also glaucoma, myocardial infarction, kidney failure and restenoses.

4. A pharmaceutical preparation comprising any one of the compounds of the formula (1) to formula (41) according to claim 1, or a pharmaceutically acceptable salt thereof, and customary excipients.

5. A pharmaceutical combination in the form of a preparation or of a kit assembled from individual components consisting a) of a compound selected from any one of the compounds of the formula (1) to formula (41) according to claim 1, or a pharmaceutically acceptable salt thereof, and b) at least one pharmaceutical dosage form whose active ingredient has cardiovascular action.

## Patentansprüche

1. Verbindung der Formel (1) 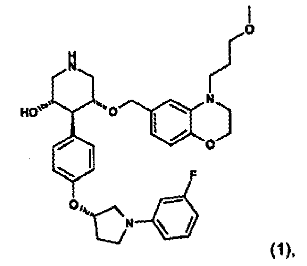 Verbindung der Formel (2) 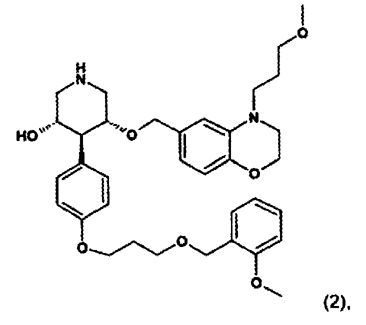 Verbindung der Formel (3) 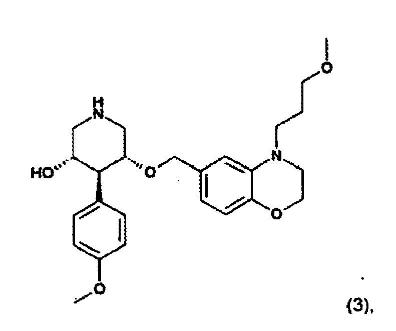 Verbindung der Formel (4) 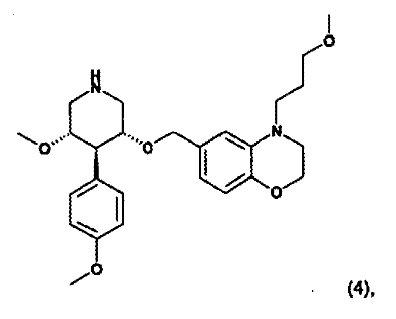 Verbindung der Formel (5) 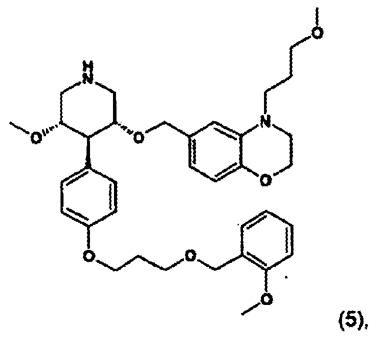 Verbindung der Formel (6) 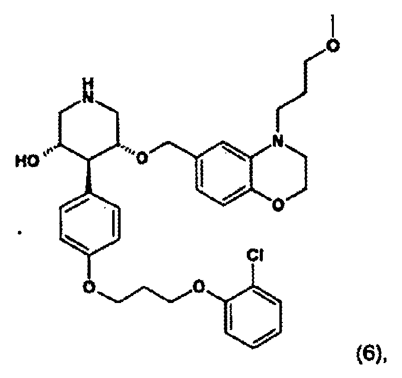 Verbindung der Formel (7) 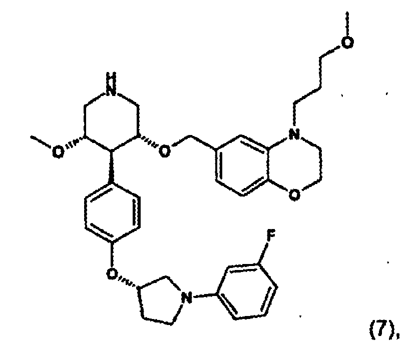 Verbindung der Formel (8) 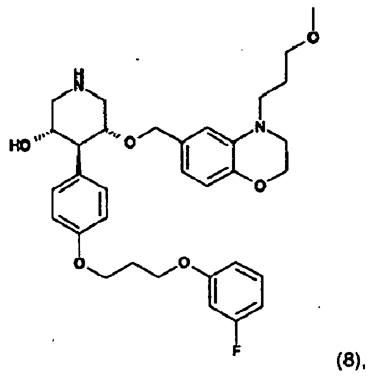 Verbindung der Formel (9) 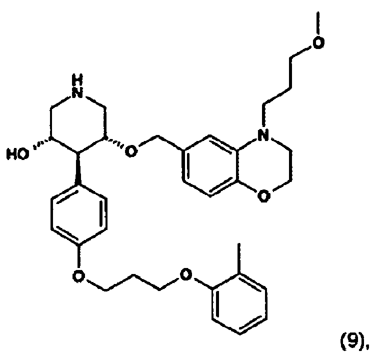 Verbindung der Formel (10) 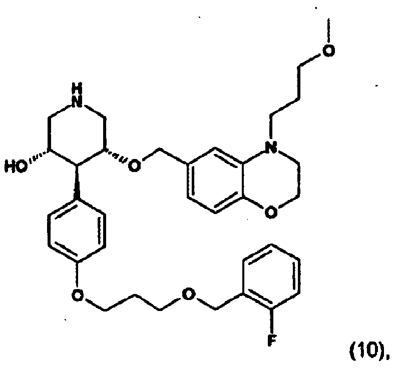 Verbindung der Formel (11) 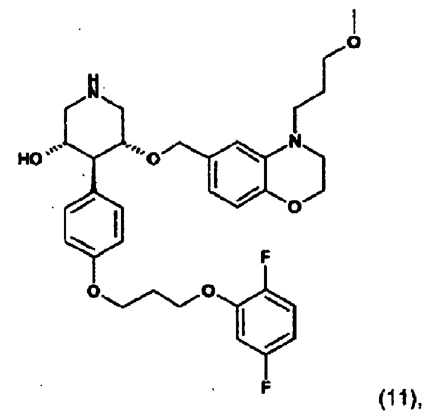 Verbindung der Formel (12) 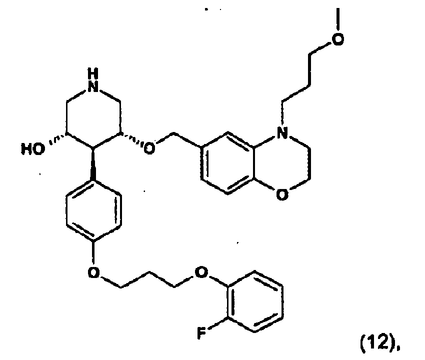 Verbindung der Formel (13) 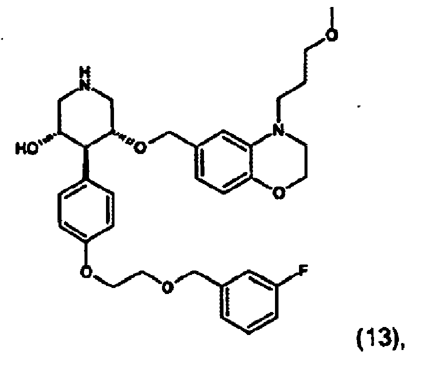 Verbindung der Formel (14) 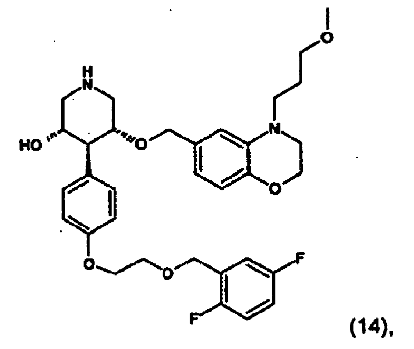 Verbindung der Formel (15) 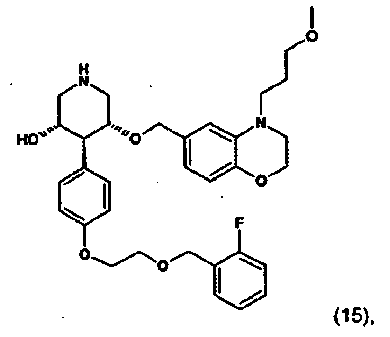 Verbindung der Formel (16) 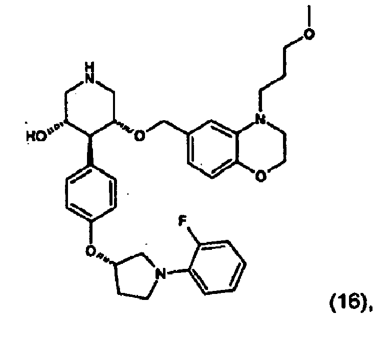 Verbindung der Formel (17) 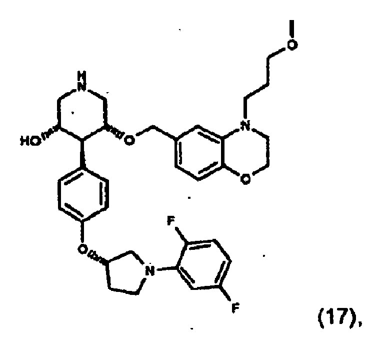 Verbindung der Formel (18) 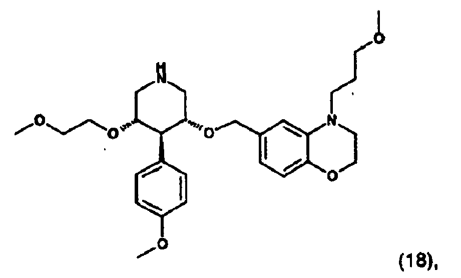 Verbindung der Formel (19) 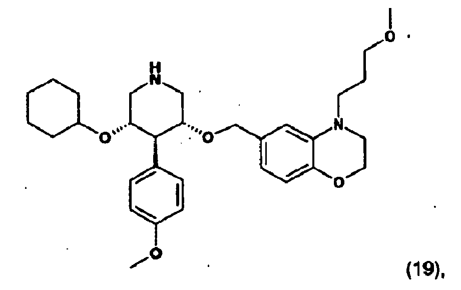 Verbindung der Formel (20) 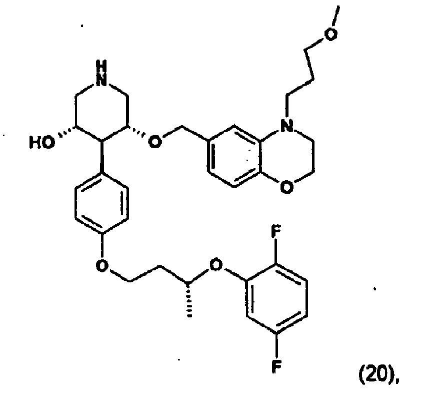 Verbindung der Formel (21) 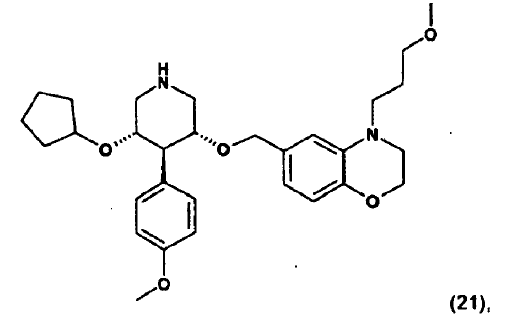 Verbindung der Formel (22) 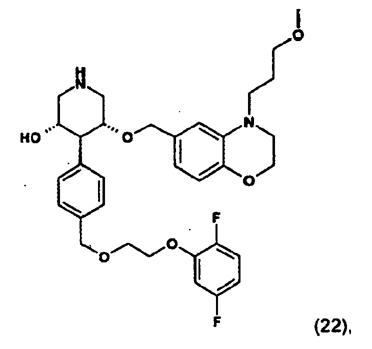 Verbindung der Formel (23) 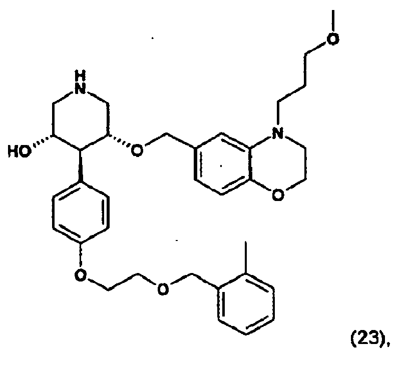 Verbindung der Formel (24) 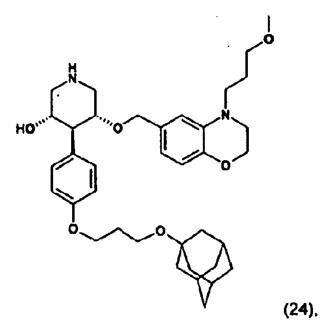 Verbindung der Formel (25) 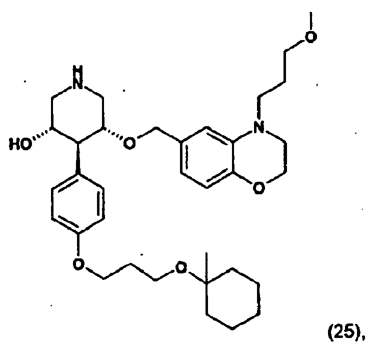 Verbindung der Formel (26) 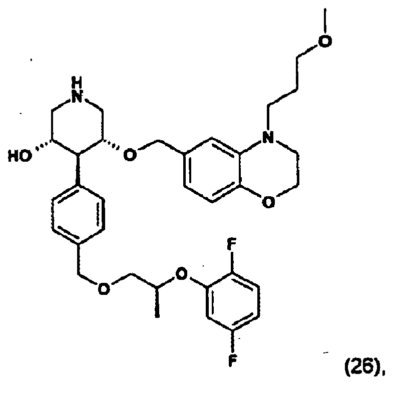 Verbindung der Formel (27) 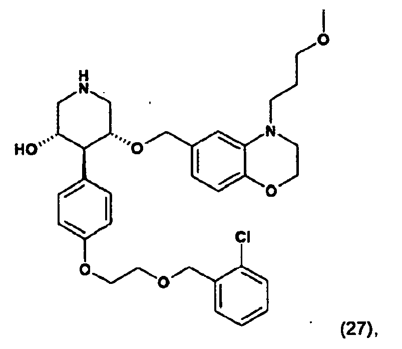 Verbindung der Formel (28) 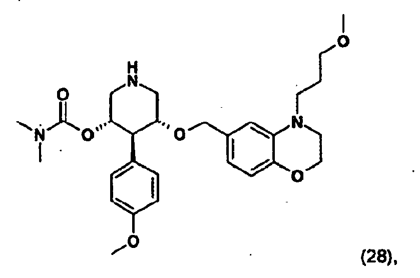 Verbindung der Formel (29) 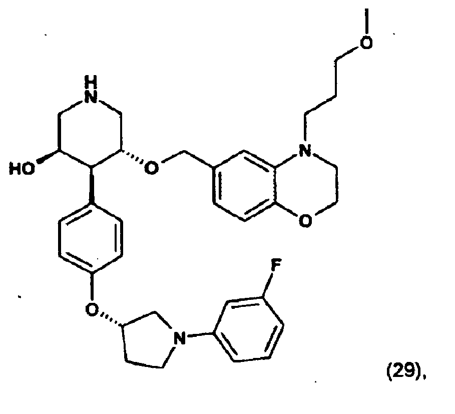 Verbindung der Formel (30) 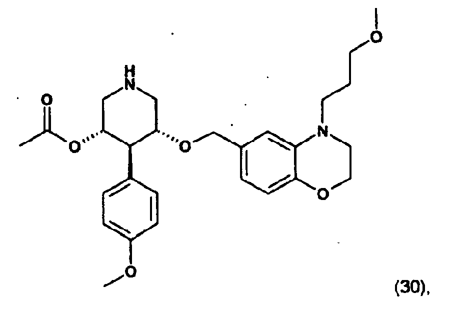 Verbindung der Formel (31) 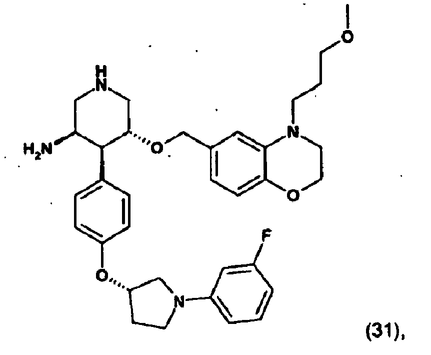 Verbindung der Formel (32) 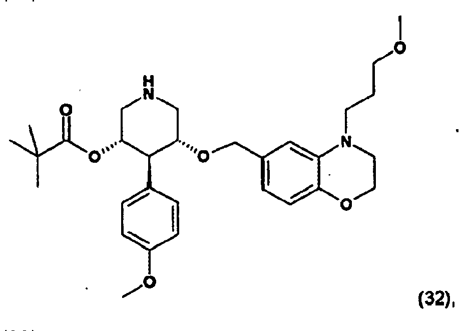 Verbindung der Formel (33) 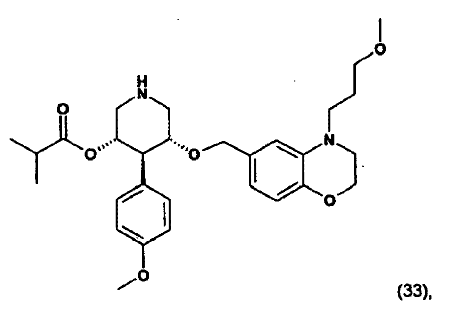 Verbindung der Formel (34) 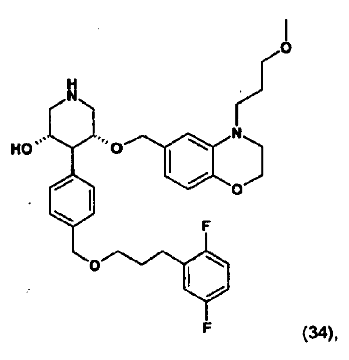 Verbindung der Formel (35) 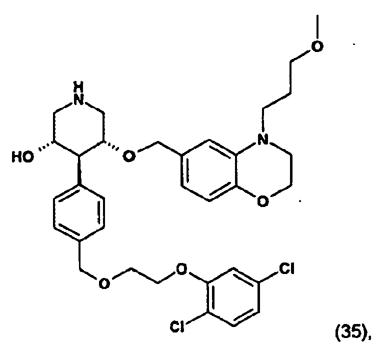 Verbindung der Formel (36) 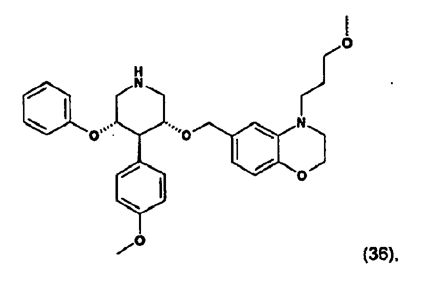 Verbindung der Formel (37) 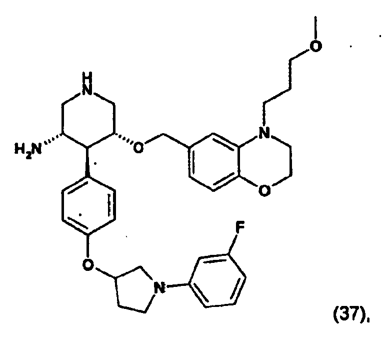 Verbindung der Formel (38) 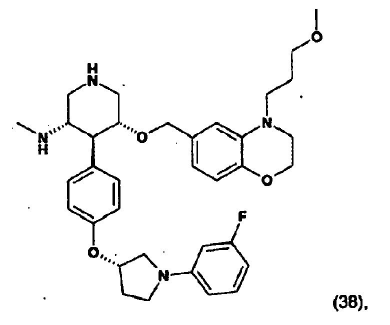 Verbindung der Formel (39) 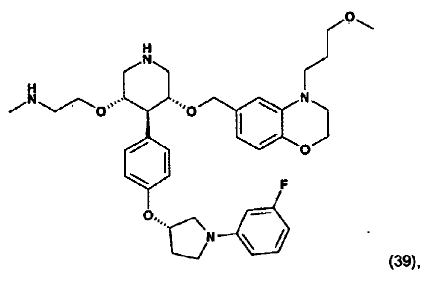 Verbindung der Formel (40) 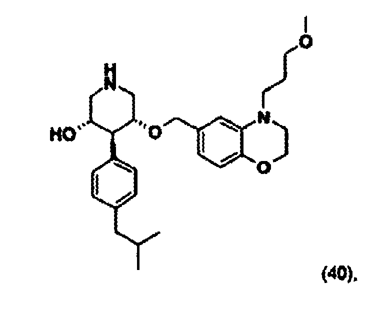 Verbindung der Formel (41) 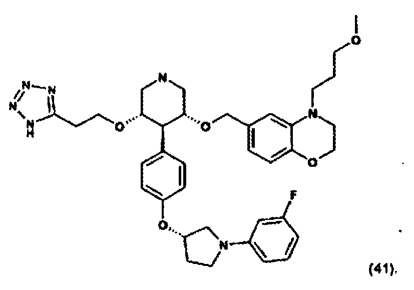 oder ein pharmazeutisch akzeptables Salz hiervon.

2. Beliebige der Verbindungen der Formeln (1) bis (41) nach Anspruch 1 oder ein pharmazeutisch akzeptables Salz hiervon zur Herstellung einer Medizin.

3. Beliebige der Verbindungen der Formeln (1) bis (41) nach Anspruch 1 oder ein pharmazeutisch akzeptables Salz hiervon zur Verwendung bei der Herstellung einer Humanmedizin zur Verhinderung, zur Verzögerung des Fortschreitens oder zur Behandlung von Bluthochdruck und Herzversagen und auch von Glaukom, Myokardinfarkt, Nierenversagen und Restenosen.

4. Pharmazeutische Zubereitung, die eine beliebige der Verbindungen der Formeln (1) bis (41) nach Anspruch 1 oder ein pharmazeutisch akzeptables Salz hiervon und herkömmliche Hilfsstoffe umfasst.

5. Pharmazeutische Kombination in Form einer Zubereitung oder eines Kits aus individuellen Komponenten, die aus a) einer Verbindung, die aus einer beliebigen der Verbindungen der Formeln (1) bis (41) nach Anspruch 1 oder einem pharmazeutisch akzeptablen Salz hiervon ausgewählt ist, und b) mindestens einer pharmazeutischen Dosierungsform bestehen, deren wirksamer Bestandteil eine kardiovaskuläre Wirkung aufweist.

## Revendications

1. Composé de formule (1) 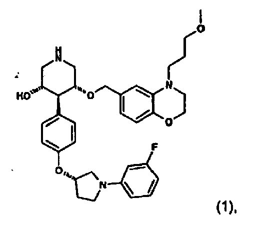 composé de formule (2) 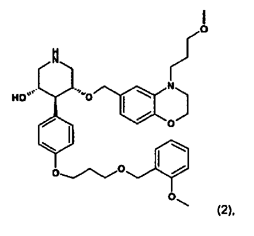 composé de formule (3) 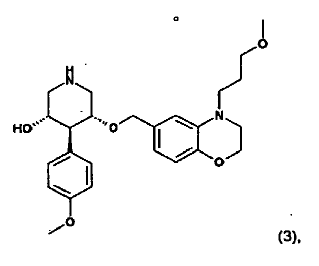 composé de formule (4) 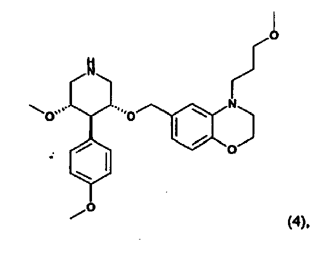 composé de formule (5) 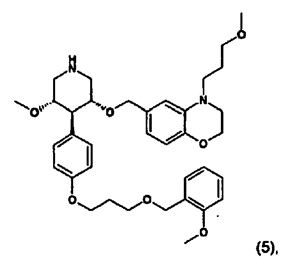 composé de formule (6) 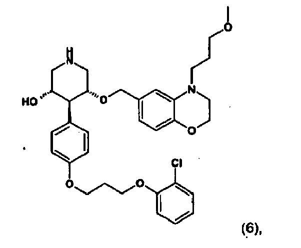 composé de formule (7) 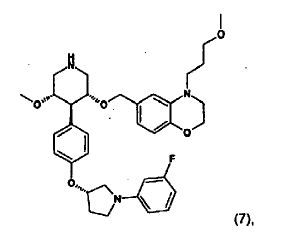 composé de formule (8) 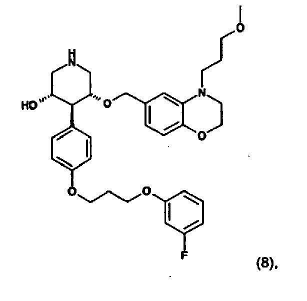 composé de formule (9) 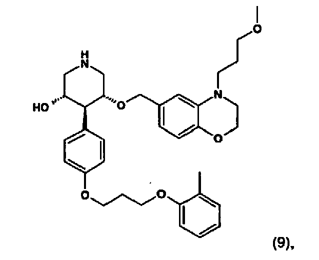 composé de formule (10) 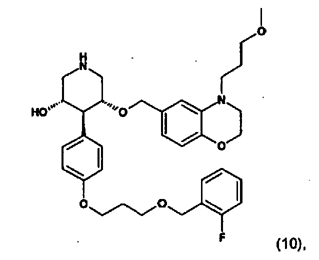 composé de formule (11) 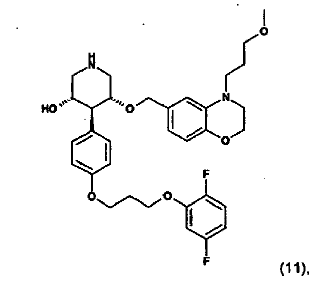 composé de formule (12) 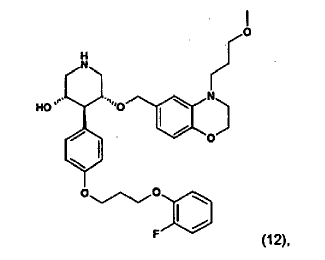 composé de formule (13) 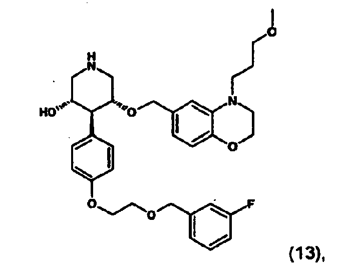 composé de formule (14) 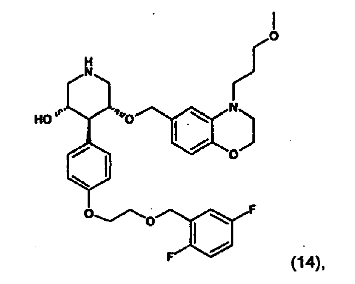 composé de formule (15) 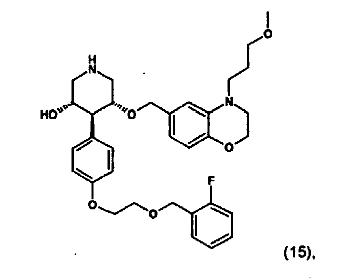 composé de formule (16) 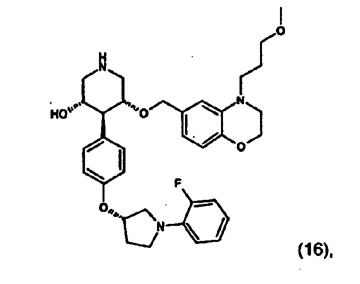 composé de formule (17) 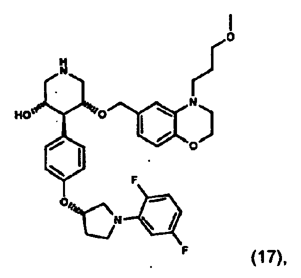 composé de formule (18) 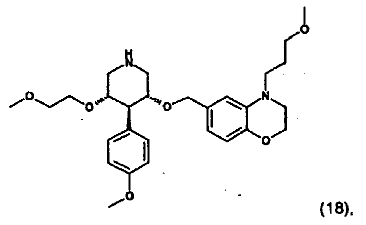 composé de formule (19) 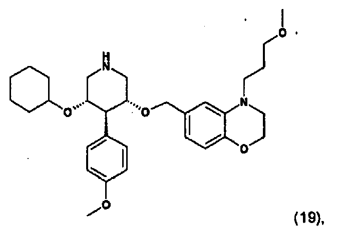 composé de formule (20) 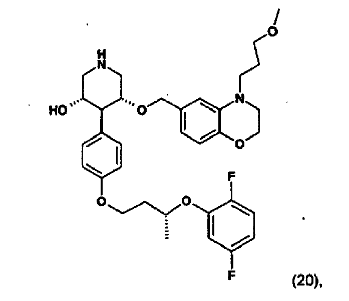 composé de formule (21) 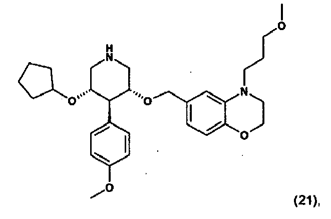 composé de formule (22) 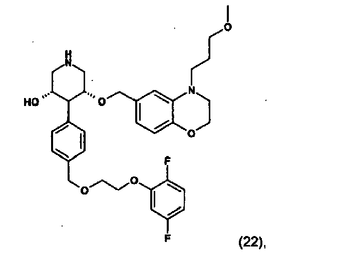 composé de formule (23) 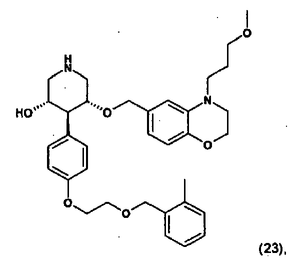 composé de formule (24) 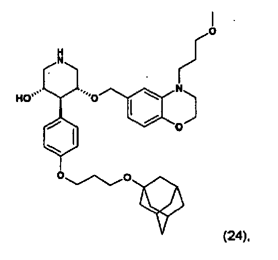 composé de formule (25) 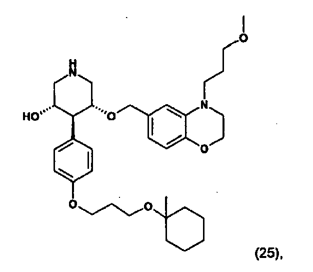 composé de formule (26) 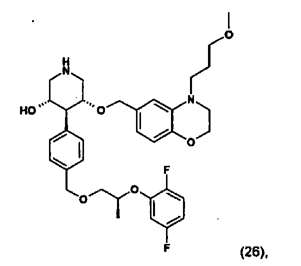 composé de formule (27) 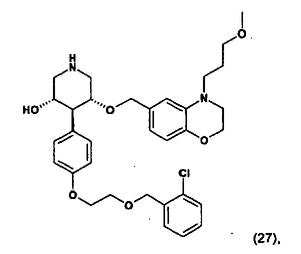 composé de formule (28) 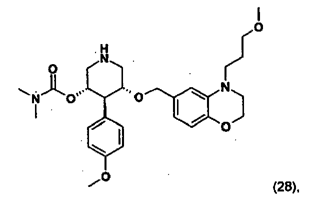 composé de formule (29) 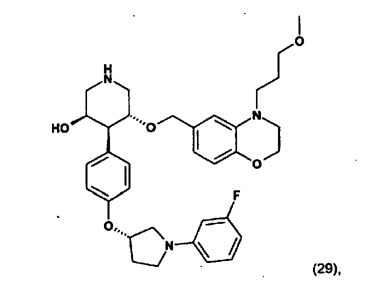 composé de formule (30) 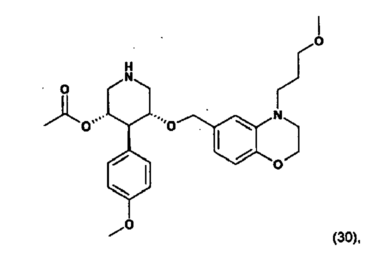 composé de formule (31) 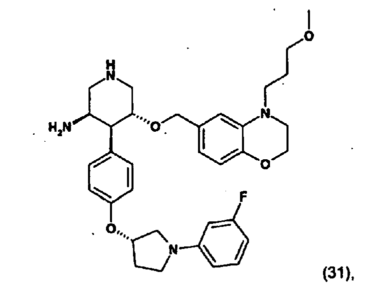 composé de formule (32) 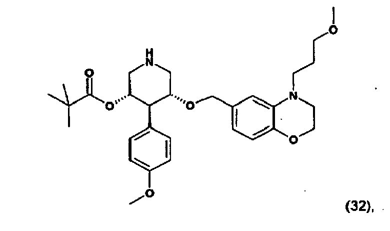 composé de formule (33) 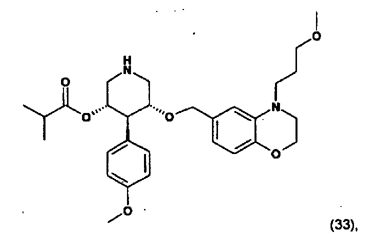 composé de formule (34) 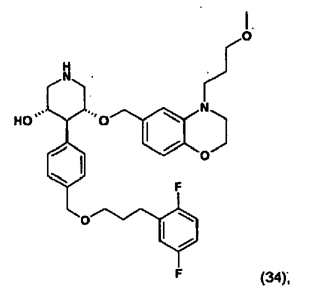 composé de formule (35) 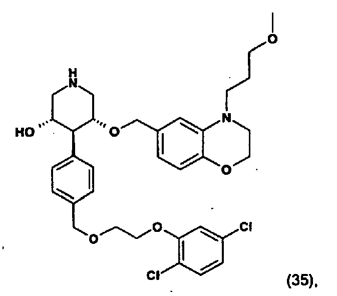 composé de formule (36) 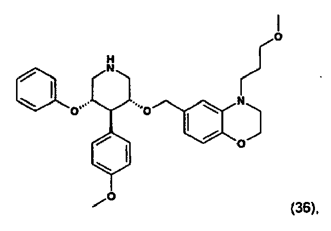 composé de formule (37) 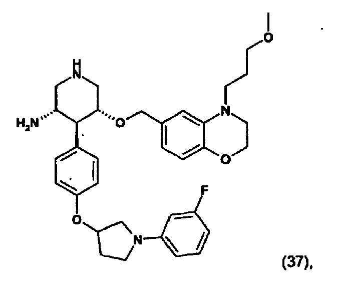 composé de formule (38) 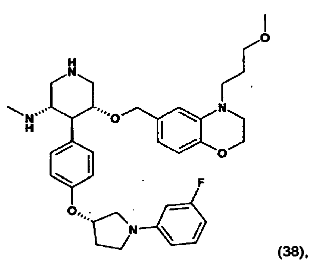 composé de formule (39) 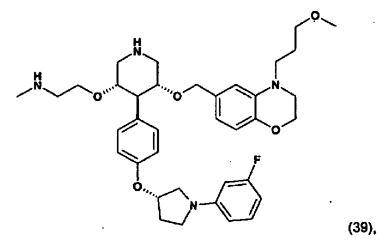 composé de formule (40), 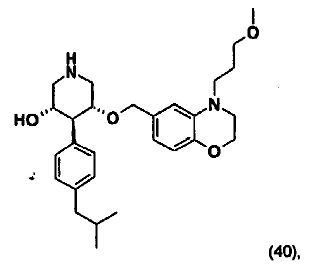 composé de formule (41) 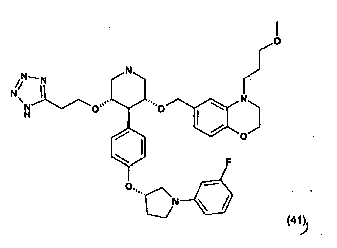 ou sel de celui-ci acceptable sur le plan pharmaceutique.

2. Composé quelconque de formule (1) à formule (41) selon la revendication 1, ou sel de celui-ci acceptable sur le plan pharmaceutique, pour produire un médicament.

3. Composé quelconque de formule (1) à formule (41) selon la revendication 1, ou sel de celui-ci acceptable sur le plan pharmaceutique, à utiliser pour produire un médicament pour humain destiné à la prévention, au retard de la progression ou au traitement de l'hypertension et de l'insuffisance cardiaque, et aussi du glaucome, de l'infarctus du myocarde, de l'insuffisance rénale et des resténoses.

4. Préparation pharmaceutique comprenant l'un quelconque des composés de formule (1) à formule (41) selon la revendication 1, ou un sel de celui-ci acceptable sur le plan pharmaceutique, et des excipients usuels.

5. Combinaison pharmaceutique sous la forme d'une préparation ou d'un kit de composants individuels assemblés composé de a) un composé sélectionné parmi l'un quelconque des composés de formule (1) à formule (41) selon la revendication 1, ou un sel de celui-ci acceptable sur le plan pharmaceutique, et b) au moins une forme posologique pharmaceutique dont l'ingrédient actif a une action cardiovasculaire.
